# EUROPEAN PATENT APPLICATION

(11) **EP 0 692 483 A1**
(43) Date of publication of application: **17.01.1996**
(21) Application number: 94910551.4
(22) Date of filing: 28.03.1994
(51) Int. Cl.: C07D 495/14, A61K 31/55, C07D 495/04

(54) **CELL ADHESION INHIBITOR AND THIENOTRIAZOLODIAZEPINE COMPOUND**

(30) Priority: 30.03.1993 JP 95398/93; 09.08.1993 JP 218195/93
(71) Applicant: YOSHITOMI PHARMACEUTICAL INDUSTRIES, LTD., Osaka-shi Osaka 541 (JP)
(72) Inventor: MORIWAKI, Minoru, Yoshitomi Pharmaceutical Ind.ltd, Osaka-shi, Osaka 541 (JP); KITANI, Hiroyuki, Yoshitomi Pharmaceut. Ind. Ltd, Chikujo-gun, Fukuoka 871 (JP); EHARA, Syuji, Yoshitomi Pharmaceutical Ind. Ltd, Chikujo-gun, Fukuoka 871 (JP); KOMATSU, Hirotsugu, Yoshitomi Pharmaceut. Ind. Ltd, Iruma-shi, Saitama 358 (JP); AMANO, Mariko, Yoshitomi Pharmaceut. Ind. Ltd., Iruma-shi, Saitama 358 (JP)
(74) Representative: von Kreisler, Alek, Dipl.-Chem.
(86) International application number: PCT/JP94/00500
(87) International publication number: WO 94/22872

(57) **Abstract**

A thienotriazolodiazepine compound such as N-(4-(4-chlorophenyl)-2,3,9-trimethyl-6H-thieno[3,2-f][1,2,4]triazolo[4,3-a][1,4]diazepin-6-yl))-N'-(2-methoxyphenyl)urea, an optically active isomer or a pharmaceutically acceptable salt thereof, and a cell adhesion inhibitor comprising said compound as the active ingredient. This compound has the effect of inhibiting cell adhesion and hence is expected to be useful in the prevention and treatment of, for example, various inflammatory diseases, allergic diseases and articular rheumatism in the onset and progress of which cell adhesion participates. Further, it is usable for preventing and treating autoimmune diseases and rejection reactions accompanying organ transplantation and, furthermore, for preventing the metastasis of tumor cells.

## Description

### Field of the Invention

The present invention relates to cell adhesion inhibitors containing a thienodiazepine compound as an active ingredient, and novel thienotriazolodiazepine compounds having inhibitory effect on cell adhesion.

### Background Art

Japanese Patent Unexamined Publication No. 223290/1991 discloses thienodiazepine compounds having cholecystokinin (CCK) antagonistic action or gastrin antagonistic action.

In various inflammatory and allergic diseases, infiltration of so-called inflammatory cells, leukocytes in a wide sense, such as polymorphonuclear leukocytes, macrophages and lymphocytes is deeply concerned with the symptoms of the diseases. Although the adhesion of leukocytes to vascular endothelial cells has been considered a first step of infiltration of leukocytes from earlier days, its mechanism has been uncertain. The recent progress in molecular biology has enabled identification of the molecules concerned with the adhesion, and their functional significance has been clarified, that is, the phenomenon of adhesion between certain adhesion molecules expressed on vascular endothelial cells and leukocytes via adhesion molecules on the leukocytes, which adhesion molecules on the leukocytes specifically binding to the certain adhesion molecules, followed by infiltration of leukocytes into inflammatory sites. While the combinations, as shown in Table 1 below, of molecules involved in the adhesion of leukocytes to vascular endothelial cells are considered to be particularly important, the degree of the involvement of respective adhesion molecules in various inflammatory and allergic diseases is not entirely clear.

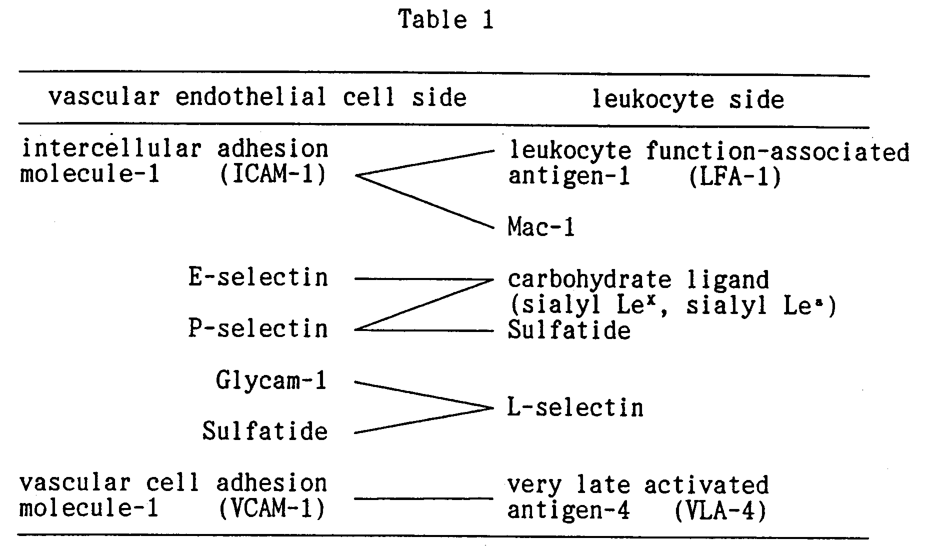

Of these adhesion molecules, ICAM-1 and LFA-1 are known to be deeply involved in not only cell infiltration, but also adhesion of lymphocytes together and their activation. For example, LFA-1 on T lymphocytes binds with ICAM-1 on macrophages to activate T lymphocytes. Accordingly, a substance which inhibits the binding between ICAM-1 and LFA-1 is expected to show immunosuppressive action in a wide sense.

In connection with diseases, promoted expression of ICAM-1 and E-selectin in inflammatory sites in inflammatory skin diseases (e.g. contact dermatitis and light eruptions caused by high photosensitivity) and rheumatoid arthritis has been reported, and involvement of ICAM-1 and Mac-1 in asthma has been suggested. It has also been reported that cell adhesion via ICAM-1 plays an important role in graft rejection after organ transplantation, and that adhesion molecules are concerned with tumor metastasis.

Using experimental animals, it has also been reported that expression of adhesion molecules has been promoted in various inflammatory models (e.g. delayed type hypersensitivity model and autoimmune nephritic model) and that anti-ICAM-1 antibody and anti-LFA-1 antibody inhibit inflammatory responses (e.g. adjuvant-induced arthritis, collagen-induced arthritis and so on). Moreover, the role of adhesion molecules in activation of eosinophils in monkey asthmatic models has been clarified. Furthermore, the effects of anti-LFA-1 antibody and anti-ICAM-1 antibody in mouse heterotopic heart transplantation models have been reported, suggesting the involvement of adhesion molecules in graft rejection, as in human beings.

Various steroidal agents, non-steroidal anti inflammatory agents, and inhibitors of the release of inflammation- and/or allergy-related mediators have been used as therapeutic agents for various inflammatory and allergic diseases. Of these pharmaceutical agents, steroidal agents often cause severe side-effects and other pharmaceutical agents fail to achieve sufficient therapeutic effects.

Recent studies have increasingly revealed that various cell adhesion molecules are deeply concerned with the onset and progress of some inflammatory and allergic diseases, and a compound having inhibitory action on cell adhesion is expected to be a superior antiinflammatory agent or anti-allergic agent. For example, Proc. Natl. Acad. Sci. U.S.A., vol. 88, pp 355-359 (1991) teaches that N-(fluorenyl-9-methoxycarbonyl)amino acids suppress reactions in various animal models of inflammation by inhibiting adhesion of leukocytes, and an abstract from American Federation for Clinical Research Annual Meeting, May 6, 1991, teaches that the same series of compounds inhibit leukocyte adhesion to vascular endothelial cells by inhibiting expression of adhesion molecules (CD18) on leukocytes.

However, the compounds described in various known literatures including the above-mentioned are not sufficient to afford satisfactory inhibitory effect on cell adhesion, and the development of a strong inhibitor of cell adhesion is desired.

### Disclosure of the Invention

With the aim of searching for a cell adhesion inhibitor, in particular, a compound which strongly inhibits adhesion of leukocytes to vascular endothelial cells (leukocyte adhesion inhibitor), the present inventors have established an assay system for cell adhesion by the use of human umbilical vein-derived endothelial cells (HUVEC) and a human leukemia cell line and conducted intensive studies. As a result, they have found that the compounds described in Japanese Patent Unexamined Publication No. 223290/1991 and novel compounds encompassed in the scope of the claims of said Publication but not specifically exemplified therein have a strong inhibitory effect on cell adhesion, which resulted in the completion of the invention.

That is, the present invention provides the following.
1. Cell adhesion inhibitors comprising, as an active ingredient, a thienodiazepine compound of the formula 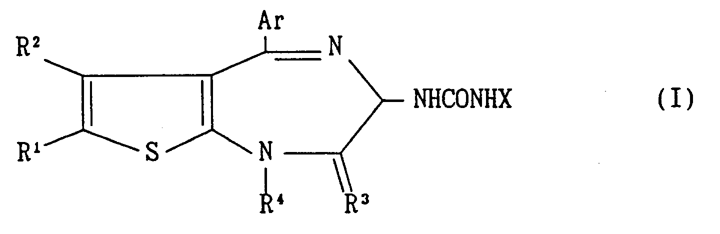 wherein
   - R¹ and R²: are the same or different, and each is a hydrogen, a halogen, an alkyl or an aralkyl, or R¹ and R² bind with each other to form a ring;
   - R³: is an oxygen;
   - R⁴: is a hydrogen, an alkyl, an alkenyl or a group of the formula: -(CH₂)mCOOR⁶ where R⁶ is a hydrogen, an alkyl, an alkenyl or an aralkyl, and m is an integer of 1 to 6, or
   - R³ and R⁴: bind with each other to form a group of the formula: =N-N=C(R⁵)- where R⁵ is a hydrogen, an alkyl, an alkenyl or an aralkyl, or a group of the formula: -(CH₂)nCOOR⁷ where R⁷ is a hydrogen, an alkyl, an alkenyl or an aralkyl and n is an integer of 1 to 6; and
   - Ar and X: are the same or different and each is an aryl, an aralkyl or a heteroaryl,
   or a pharmaceutically acceptable salt thereof, methods for treating various diseases concerned with cell adhesion, comprising administering said compound, and use of said compound for producing cell adhesion inhibitors.
2. Thienodiazepine compounds of the formula 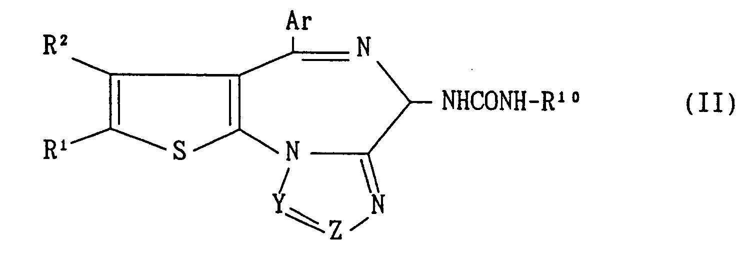 wherein
   - Ar: is a phenyl, a pyridyl, a phenyl having, as a substituent, halogen, alkyl having 1 to 4 carbon atoms, alkoxy having 1 to 4 carbon atoms, cyano, amino, nitro or trihalomethyl, or a pyridyl having, as a substituent, halogen, alkyl having 1 to 4 carbon atoms or alkoxy having 1 to 4 carbon atoms;
   - R¹ and R²: are the same or different and each is a hydrogen, a halogen or an alkyl, or R¹ and R² bind with each other to form a ring;
   - -Y=Z-: is a group of the formula: -N=N-, -C(R⁵)=N- where R⁵ is a hydrogen, a halogen, an alkyl, a haloalkyl or a group of the formula: -(CH₂)nNR¹¹R¹² where R¹¹ and R¹² are the same or different and each is a hydrogen, an alkyl having 1 to 4 carbon atoms, or an alkenyl having 2 to 8 carbon atoms or an aralkyl and n is an integer of 1 to 6, or -CH=CH-; and
   - R¹⁰: is a phenyl, a pyridyl or a phenyl having 1 to 3 substituents selected from halogen, amino, nitro, trihalomethyl, alkyl having 1 to 4 carbon atoms and alkoxy having 1 to 4 carbon atoms,
   optically active compounds thereof and pharmaceutically acceptable salts thereof.
   In the above definitions and in the present specification, halogen means chlorine, bromine, fluorine and iodine. Alkyl means alkyl having 1 to 20 carbon atoms, such as methyl, ethyl, propyl, isopropyl, butyl, isobutyl, tert-butyl, pentyl, isopentyl, tert-pentyl, hexyl, octyl, 2-ethylhexyl, 1,1,3,3-tetramethylbutyl, nonyl, decyl, dodecyl, tetradecyl, octadecyl and icocyl, with preference given to alkyl having 1 to 4 carbon atoms, particularly specifically, methyl. Alkenyl has 2 to 8 carbon atoms and includes, for example, vinyl, 1-propenyl, allyl, isopropenyl, 2-butenyl, 2-pentenyl, 3-hexenyl and 6-octenyl. Aralkyl may have, on its aromatic ring, 1 to 3 substituents selected from halogen, alkyl, alkoxy, trifluoromethyl, nitro, amino, cyano and hydroxy, and is exemplified by benzyl, 1-phenylethyl, 2-phenylethyl, 3-phenylpropyl, 4-phenylbutyl, 1-naphthylmethyl, 2-naphthylmethyl and diphenylmethyl. Aryl may have, on its aromatic ring, 1 to 3 substituents selected from halogen, alkyl, alkoxy, trifluoromethyl, nitro, amino, cyano and hydroxy, and is exemplified by phenyl, 1-naphthyl and 2-naphthyl, with particular preference given to phenyl. Heteroaryl may have, on its ring, 1 to 3 substituents selected from halogen, alkyl, alkoxy, trifluoromethyl, nitro, amino, cyano and hydroxy, and is exemplified by pyridyl (e.g. 2-pyridyl, 3-pyridyl and 4-pyridyl), quinolyl (e.g. 2-quinolyl and 3-quinolyl), indolyl (e.g. 2-indolyl and 3-indolyl), thienyl (e.g. 2-thienyl and 3-thienyl), furyl (e.g. 2-furyl and 3-furyl), benzofuranyl (e.g. 2-benzofuranyl and 3-benzofuranyl), 1H-benzimidazol-2-yl and 2-benzothiazolyl. The ring formed by R¹ and R² or R¹' and R²' which are bound with each other means cyclopentene ring, cyclopentadiene ring, cyclohexene ring, cyclohexadiene ring, benzene ring, cycloheptene ring, cycloheptadiene ring, cycloheptatriene ring and the like.
   Alkyl having 1 to 4 carbon atoms is exemplified by methyl, ethyl, propyl, isopropyl, butyl, isobutyl and tert-butyl. Alkoxy having 1 to 4 carbon atoms is exemplified by methoxy, ethoxy, propoxy, isopropoxy, butoxy, isobutoxy and tert-butoxy. Trihalomethyl means trichloromethyl, trifluoromethyl, tribromomethyl and the like. Haloalkyl means that having 1 to 4 carbon atoms, and is exemplified by chloromethyl, bromomethyl, fluoromethyl, difluoromethyl, trifluoromethyl, dichloromethyl and 2,2,2-trifluoroethyl.
   The preferable mode of the thienodiazepine compound of the formula (II) includes, for example, the compounds of the formulas (III), (IV), (V) and (VI). The compounds of the formulas (III)-(VI) have strong cell adhesion inhibitory activity but have weak CCK antagonistic action. In particular, the compounds of the formulas (V) and (VI) have weak CCK antagonistic action.
3. Thienotriazolodiazepine compounds of the formula 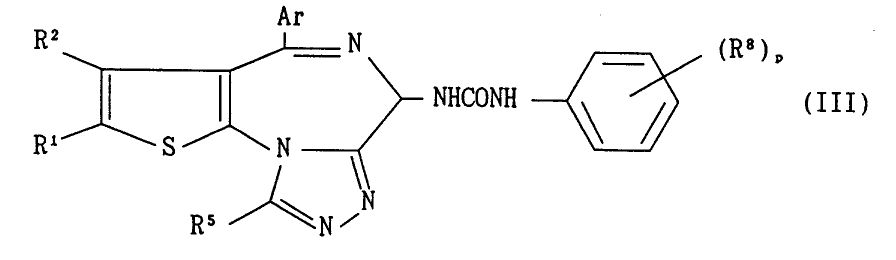 wherein
   - Ar: is a phenyl, a pyridyl, a phenyl having, as a substituent, halogen, alkyl having 1 to 4 carbon atoms, alkoxy having 1 to 4 carbon atoms, cyano, amino, nitro or trihalomethyl, or a pyridyl having, as a substituent, halogen, alkyl having 1 to 4 carbon atoms or alkoxy having 1 to 4 carbon atoms;
   - R¹ and R²: are the same or different and each is a hydrogen, a halogen or an alkyl having 1 to 4 carbon atoms, or R¹ and R² bind with each other to form a ring;
   - R⁵: is a hydrogen, a halogen or an alkyl having 1 to 4 carbon atoms;
   - R⁸: is a hydrogen, a halogen, an amino, a nitro, a trihalomethyl, an alkyl having 1 to 4 carbon atoms or an alkoxy having 1 to 4 carbon atoms; and
   - p: is 1, 2 or 3, provided that when p is 2 or 3, the groups represented by R⁸ are the same or different,
   optically active compounds thereof and pharmaceutically acceptable salts thereof.
4. Thienotriazolodiazepine compounds of the formula 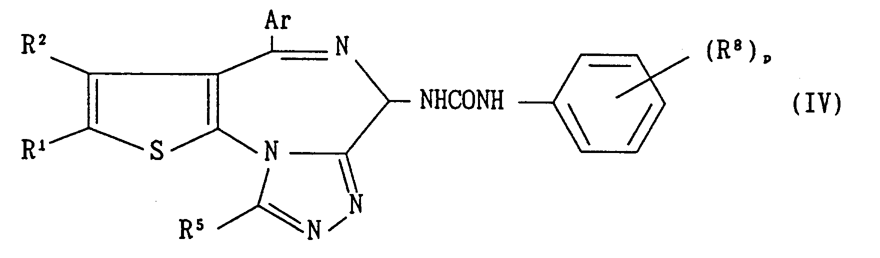 wherein
   - Ar: is a phenyl or a phenyl having, as a substituent, halogen, alkyl having 1 to 4 carbon atoms or alkoxy having 1 to 4 carbon atoms;
   - R¹: is an alkyl having 1 to 4 carbon atoms;
   - R²: is a hydrogen or an alkyl having 1 to 4 carbon atoms, or R¹ and R² bind with each other to form a 5- to 7-membered ring;
   - R⁵: is an alkyl having 1 to 4 carbon atoms;
   - R⁸: is an alkyl having 1 to 4 carbon atoms or an alkoxy having 1 to 4 carbon atoms; and
   - p: is 1 or 2, provided that when p is 2, the groups represented by R⁸ are the same or different,
   optically active compounds thereof and pharmaceutically acceptable salts thereof.
5. Thienotriazolodiazepine compounds of the formula 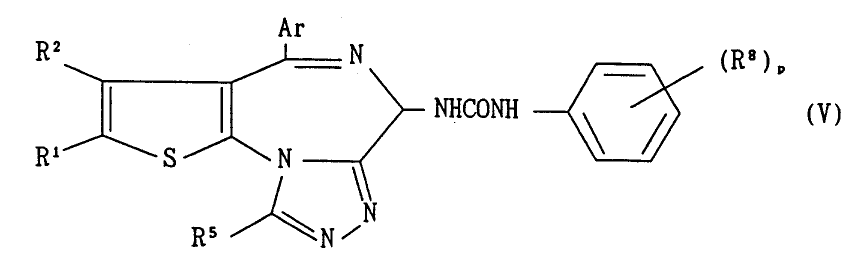 wherein
   - Ar: is a phenyl or a phenyl having, as a substituent, halogen, methyl or methoxy;
   - R¹: is a methyl or an ethyl;
   - R²: is a hydrogen or a methyl; or R¹ and R² bind with each other to form a group of the formula: -(CH₂)₃- or -(CH₂)₄-;
   - R⁵: is a methyl;
   - R⁸: is a methyl or a methoxy; and
   - p: is 1 or 2, provided that when p is 2, the groups represented by R⁸ are the same or different,
   optically active compounds thereof and pharmaceutically acceptable salts thereof.
6. Thienotriazolodiazepine compounds of the formula 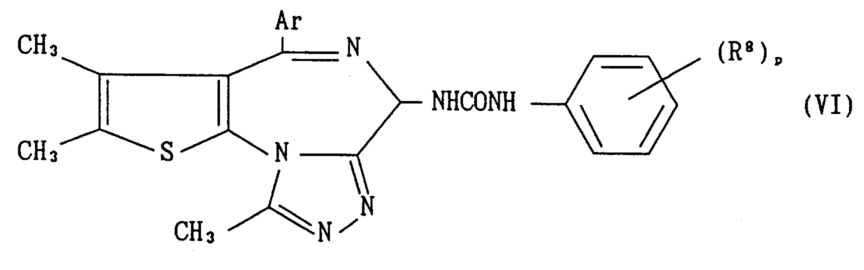 wherein
   - Ar: is a phenyl or a phenyl having, as a substituent, halogen, methyl or methoxy;
   - R⁸: is a methyl or a methoxy; and
   - p: is 1 or 2, provided that when p is 2, the groups represented by R⁸ are the same or different,
   optically active compounds thereof and pharmaceutically acceptable salts thereof.
7. Examples of the compounds encompassed in the formula (II), which have a strong cell adhesion inhibitory action, include the following compounds. These compounds scarcely show CCK antagonistic action.
Thienotriazolodiazepine compounds selected from
(1) N-4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(2) N-(2,3,9-trimethyl-4-phenyl-6H-thieno[3,2-f][1,2,4]triazolo[4,3-a][1,4]diazepin-6-yl)-N'-(2-methoxyphenyl)urea,
(3) N-(2,3,9-trimethyl-4-(4-methylphenyl)-6H-thieno[3,2-f][1,2,4]triazolo[4,3-a][1,4]diazepin-6-yl)-N'-(2-methoxyphenyl)urea,
(4) N-(4-(4-methoxyphenyl)-2,3,9-trimethyl-6H-thieno[3,2-f][1,2,4]triazolo[4,3-a][1,4]diazepin-6-yl)-N'-(2-methoxyphenyl)urea,
(5) N-(2,3,9-trimethyl-4-phenyl-6H-thieno[3,2-f][1,2,4]triazolo[4,3-a][1,4]diazepin-6-yl)-N'-(3-methoxyphenyl)urea,
(6) N-(2,3,9-trimethyl-4-phenyl-6H-thieno[3,2-f][1,2,4]triazolo[4,3-a][1-4]diazepin-6-yl)-N'-(2-methylphenyl)urea,
(7) N-(4-(4-chlorophenyl)-2,3,9-trimethyl-6H-thieno[3,2-f][1,2,4]triazolo[4,3-a][1,4]diazepin-6-yl)-N'-(3-methoxyphenyl)urea,
(8) N-(4-(4-chlorophenyl)-2,3,9-trimethyl-6H-thieno[3,2-f][1,2,4]triazolo[4,3-a][1,4]diazepin-6-yl)-N'-(2-methylphenyl)urea,
(9) N-(4-(4-bromophenyl)-2,3,9-trimethyl-6H-thieno[3,2-f][1,2,4]triazolo[4,3-a][1,4]diazepin-6-yl)-N'-(2-methoxyphenyl)urea,
(10) N-(4-(4-bromophenyl)-2,3,9-trimethyl-6H-thieno[3,2-f][1,2,4]triazolo[4,3-a][1,4]diazepin-6-yl)-N'-(3-methoxyphenyl)urea,
(11) N-(4-(4-fluorophenyl)-2,3,9-trimethyl-6H-thieno[3,2-f][1,2,4]triazolo[4,3-a][1,4]diazepin-6-yl)-N'-(2-methoxyphenyl)urea,
(12) N-(2,3,9-trimethyl-4-(4-methylphenyl)-6H-thieno[3,2-f][1,2,4]triazolo[4,3-a][1,4]diazepin-6-yl)-N'-(3-methoxyphenyl)urea,
(13) N-4-(4-methoxyphenyl)-2,3,9-trimethyl-6H-thieno[3,2-f][1,2,4]triazolo[4,3-a][1,4]diazepin-6-yl)-N'-(3-methoxyphenyl)urea,
(14) N-4-(2-chlorophenyl)-2,3,9-trimethyl-6H-thieno[3,2-f][1,2,4]triazolo[4,3-a][1,4]diazepin-6-yl)-N'-(2-methoxyphenyl)urea,
(15) N-(4-(3-chlorophenyl)-2,3,9-trimethyl-6H-thieno[3,2-f][1,2,4]triazolo[4,3-a][1,4]diazepin-6-yl)-N'-(2-methoxyphenyl) urea,
(16) N-(2,3,9-trimethyl-4-(2-methylphenyl)-6H-thieno[3,2-f][1,2,4]triazolo[4,3-a][1,4]diazepin-6-yl)-N'-(2-methoxyphenyl)urea,
(17) N-(2,3,9-trimethyl-4-(3-methylphenyl)-6H-thieno[3,2-f][1,2,4]triazolo[4,3-a][1,4]diazepin-6-yl)-N'-(2-methoxyphenyl)urea,
(18) N-(4-(2-methoxyphenyl)-2,3,9-trimethyl-6H-thieno[3,2-f][1,2,4]triazolo[4,3-a][1,4]diazepin-6-yl)-N'-(2-methoxyphenyl)urea,
(19) N-(4-(3-methoxyphenyl)-2,3,9-trimethyl-6H-thieno[3,2-f][1,2,4]triazolo[4,3-a][1,4]diazepin-6-yl)-N'-(2-methoxyphenyl)urea,
(20) (+)-N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(21) (-)-N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(22) N-(4-(2-chlorophenyl)-2,3,9-trimethyl-6H-thieno[3,2-f][1,2,4]triazolo[4,3-a][1,4]diazepin-6-yl)-N'-(3-methylphenyl)urea,
(23) N-(4-(4-chlorophenyl)-2,3,9-trimethyl-6H-thieno[3,2-f][1,2,4]triazolo[4,3-a][1,4]diazepin-6-yl)-N'-(2,5-dimethoxyphenyl)urea,
(24) N-(6-(4-chlorophenyl)-11-methyl-2,3,4,5-tetrahydro-8H-[1]benzothieno[3,2-f][1,2,4]triazolo[4,3-a][1,4]diazepin-8-yl)-N'- (2-methoxyphenyl)urea,
(25) N-(5-(4-chlorophenyl)-3,4-dihydro-2H,7H-cyclopenta[4,5]thieno[3,2-f][1,2,4]triazolo[4,3-a][1,4]diazepin-7-yl)-N'-(2-methoxyphenyl)urea,
(26) N-(4-(4-chlorophenyl)-2-ethyl-3,9-dimethyl-6H-thieno[3,2-f][1,2,4]triazolo[4,3-a][1,4]diazepin-6-yl)-N'-(2-methoxyphenyl)urea, and
(27) N-(4-(4-chlorophenyl)-2,9-dimethyl-6H-thieno-[3,2-f][1,2,4]triazolo[4,3-a][1,4]diazepin-6-yl)-N'-(2-methoxyphenyl)urea,
optically active compounds thereof and pharmaceutically acceptable salts thereof.

Of the above-mentioned, (-)-N-4-(4-chlorophenyl)-2,3,9-trimethyl-6H-thieno[3,2-f][1,2,4]triazolo[4,3-a][1,4]diazepin-6-yl)-N'-(2-methoxyphenyl)urea and pharmaceutically acceptable salts thereof are preferable in terms of strong cell adhesion inhibitory action.

In addition, the mode of the thienodiazepine compound of the formula (II) include the compounds of the formula (VII) 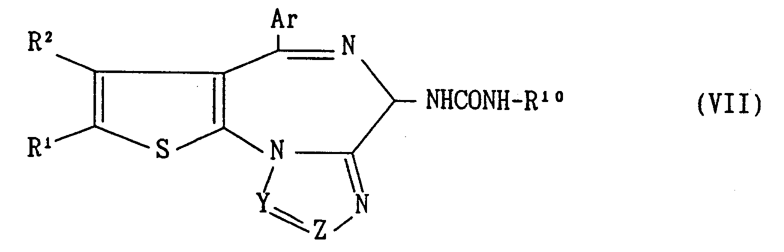
wherein
- Ar: is a phenyl, a pyridyl, a phenyl having, as a substituent, halogen, alkyl having 1 to 4 carbon atoms, alkoxy having 1 to 4 carbon atoms, cyano, amino, nitro or trihalomethyl, or a pyridyl having, as a substituent, halogen, alkyl having 1 to 4 carbon atoms or alkoxy having 1 to 4 carbon atoms;
- R¹ and R²: are the same or different and each is a hydrogen, a halogen or an alkyl having 1 to 4 carbon atoms, or R¹ and R² bind with each other to form a ring;
- -Y=Z-: is a group of the formula: -N=N-, -C(R⁵)=N- where R⁵ is a haloalkyl or a group of the formula: -(CH₂)nNR¹¹R¹² where R¹¹ and R¹² are the same or different and each is a hydrogen, an alkyl having 1 to 4 carbon atoms, or an alkenyl having 2 to 8 carbon atoms or an aralkyl, and n is an integer of 1 to 6, or -CH=CH-; and
- R¹⁰: is a phenyl, a pyridyl or a phenyl having 1 to 3 substituents selected from halogen, amino, nitro, trihalomethyl, alkyl having 1 to 4 carbon atoms and alkoxy having 1 to 4 carbon atoms,
optically active compounds thereof and pharmaceutically acceptable salts thereof.

Ar in the above formulas (I) - (VII) is preferably a phenyl having, at the 4-position, a substituent such as halogen, methyl and methoxy.

The pharmaceutically acceptable salts of the compounds of the formulas (I) and (II) include, for example, addition salts with inorganic acid or organic acid, and salts with inorganic base, organic base or amino acid, and preferred in view of the object of the present invention are the salts substantially non-toxic. The compounds of the formulas (I) and (II) have at least one chiral carbon atom. The racemates, optically active compounds and diastereomers are all encompassed in the present invention.

The compound of the formula (I) can be produced by the method described in Japanese Patent Unexamined Publication No. 223290/1991. The compound of the formula (II) can be produced by, for example, condensing a compound of the formula 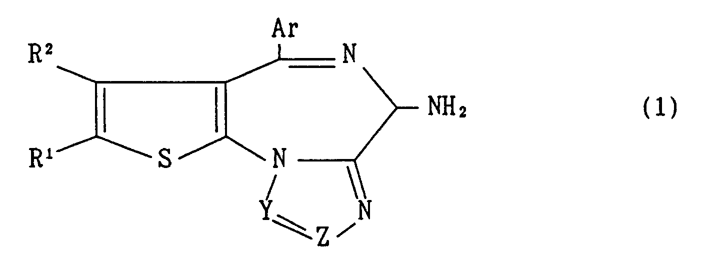
wherein each symbol is as defined above, with isocyanate of the formula

R¹⁰-N=C=O (2)

wherein R¹⁰ is as defined above.

The condensation reaction of a compound of the formula (1) and a compound of the formula (2) is carried out in a suitable solvent which does not interfere with the instant reaction. Examples of the solvent include organic solvents such as tetrahydrofuran, diethyl ether, diisopropyl ether, dioxane, dichloromethane, chloroform, ethyl acetate, benzene, toluene, xylene, dimethylformamide and dimethylacetamide.

The temperature of the condensation reaction varies depending on the reagent and solvent to be used. Preferable temperature is generally from -20°C to the boiling point of the solvent.

The starting compound of the formula (1) can be produced by, for example, reacting a compound of the formula 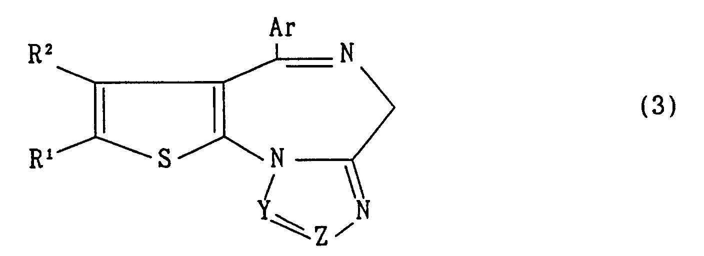
wherein each symbol is as defined above, with a dialkyl carbonate such as diethyl carbonate in the presence of a base such as sodium hydride and potassium tert-butoxy to introduce alkoxycarbonyl such as ethoxycarbonyl at the 6-position, reacting the resulting compound with O-(2,4-dinitrophenyl)hydroxylamine to give a compound of the formula 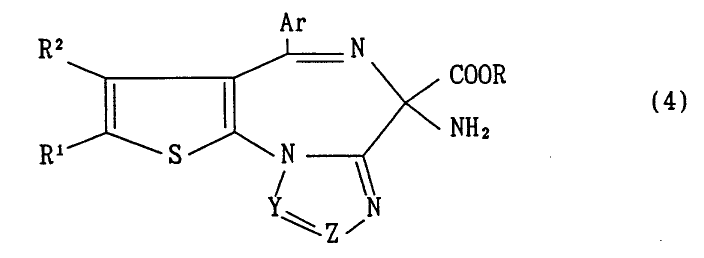
wherein R is an alkyl and other symbols are as defined above, subjecting this compound of the formula (4) to hydrolysis in water or a mixed solvent of water and organic solvent which is preferably methanol, ethanol, diethyl ether, tetrahydrofuran or dioxane, in the presence of a base such as sodium hydroxide, potassium hydroxide and barium hydroxide at a temperature of from about 0°C to the boiling point of the solvent used, and making the obtained reaction mixture assume acidity using an acid such as hydrochloric acid, hydrobromic acid, hydrofluoric acid, sulfuric acid, trifluoroacetic acid and trifluoromethanesulfonic acid. The compound of the formula (3) can be synthesized by the method described in Japanese Patent Publication No. 43477/1980.

The thus obtained compound of the formula (I) or (II) can be separated from the reaction mixture or purified by a method known *per se* such as recrystallization and column chromatography.

The compound of the formula (I) or (II) can be converted to salts by treating same with an inorganic acid such as hydrochloric acid, hydrobromic acid, sulfuric acid, phosphoric acid and nitric acid, an organic acid such as acetic acid, propionic acid, succinic acid, glycolic acid, lactic acid, malic acid, tartaric acid, citric acid, maleic acid, fumaric acid, methanesulfonic acid, benzenesulfonic acid and ascorbic acid, an inorganic base such as sodium hydroxide, potassium hydroxide, calcium hydroxide, magnesium hydroxide, zinc hydroxide and ammonium hydroxide, an organic base such as methylamine, diethylamine, triethylamine, dicyclohexylamine, triethanolamine, ethylenediamine, trishydroxymethylaminomethane, quinine, guanidine and cinchonine, or an amino acid such as lysine, ornithine, arginine and alanine by a conventional method.

Of the compounds of the compounds of the present invention, when a compound has a chiral carbon atom, it is generally obtained as a racemate. The racemate can be resolved into optical isomers by a conventional method such as high performance liquid chromatography (HPLC) using packed optical isomer separation column. Such optical isomers can be also produced by using an optically active starting material. Respective diastereomers can be purified by separation crystallization and chromatography.

Examples of the compounds encompassed in the formulas (I) and (II) are as shown in the following Tables. In the Tables, Cl means chlorine, Et means ethyl, Me means methyl, MeO means methoxy, NO₂ means nitro, Ph means phenyl, 4-Py means 4-pyridyl, 2-Py means 2-pyridyl, and 4-MeOPh means, for example, 4-methoxyphenyl.

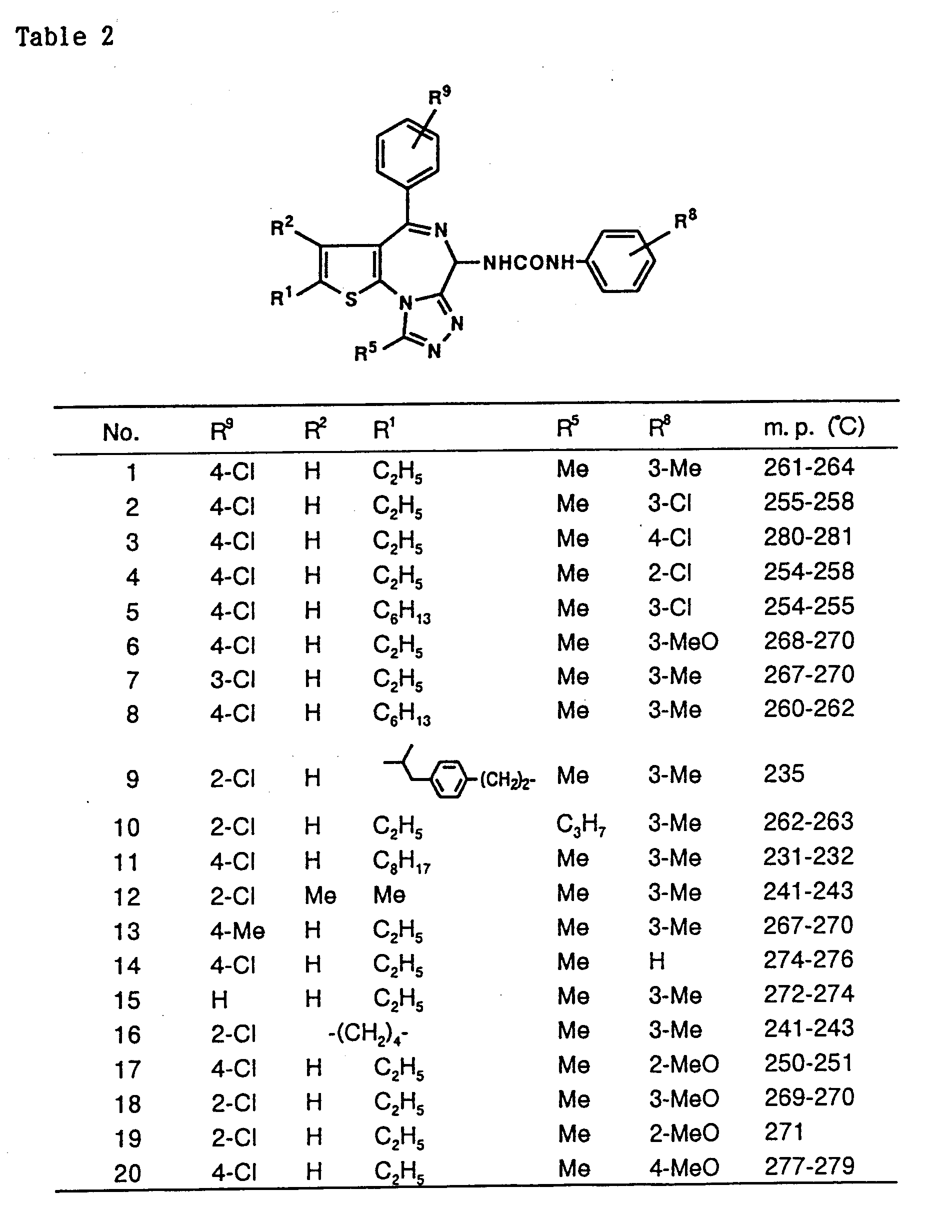

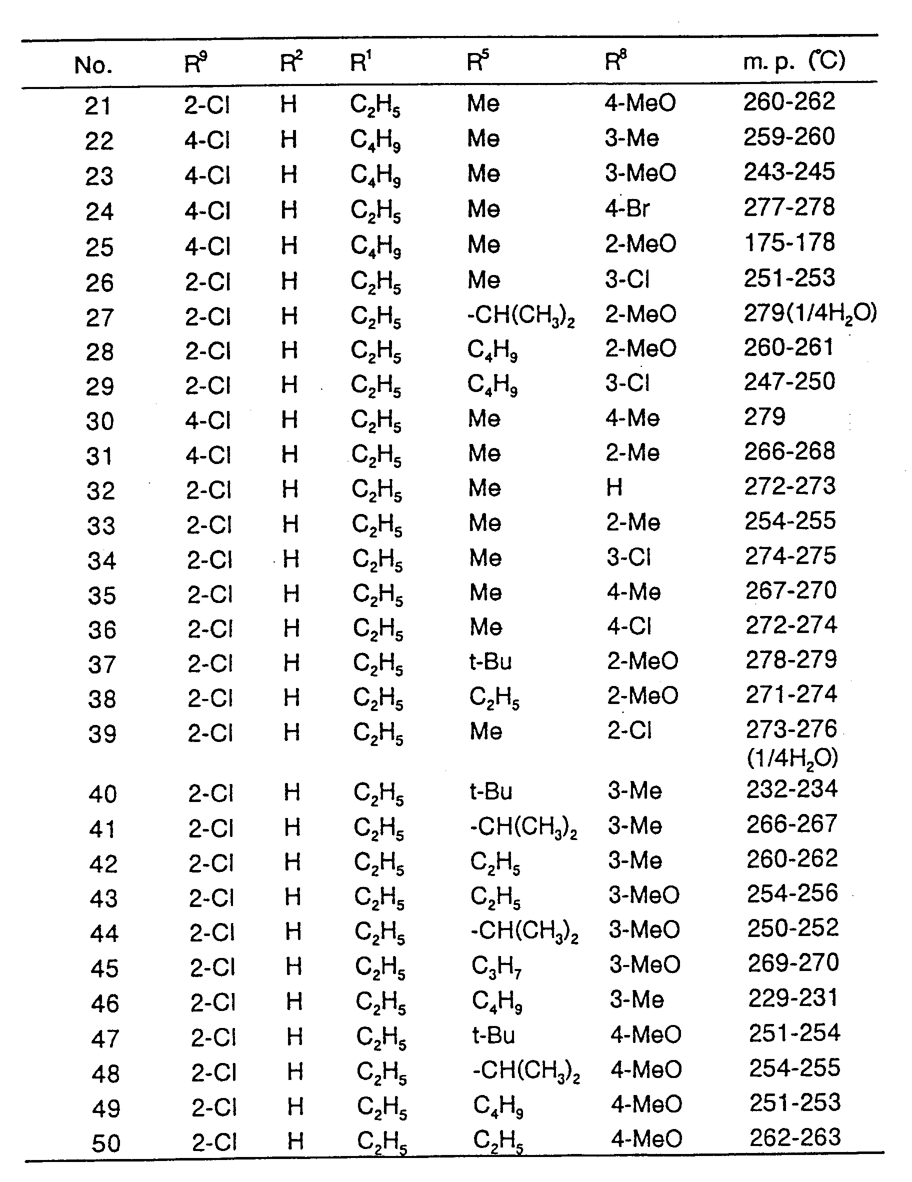

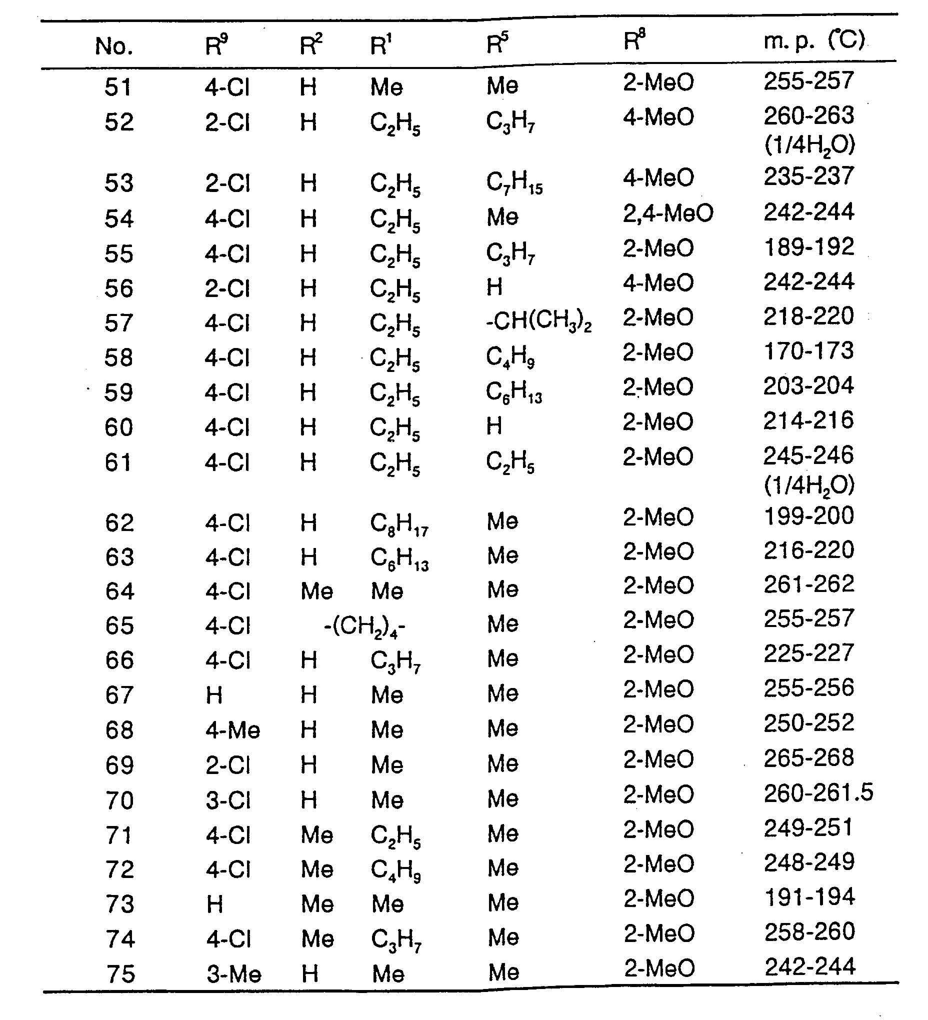

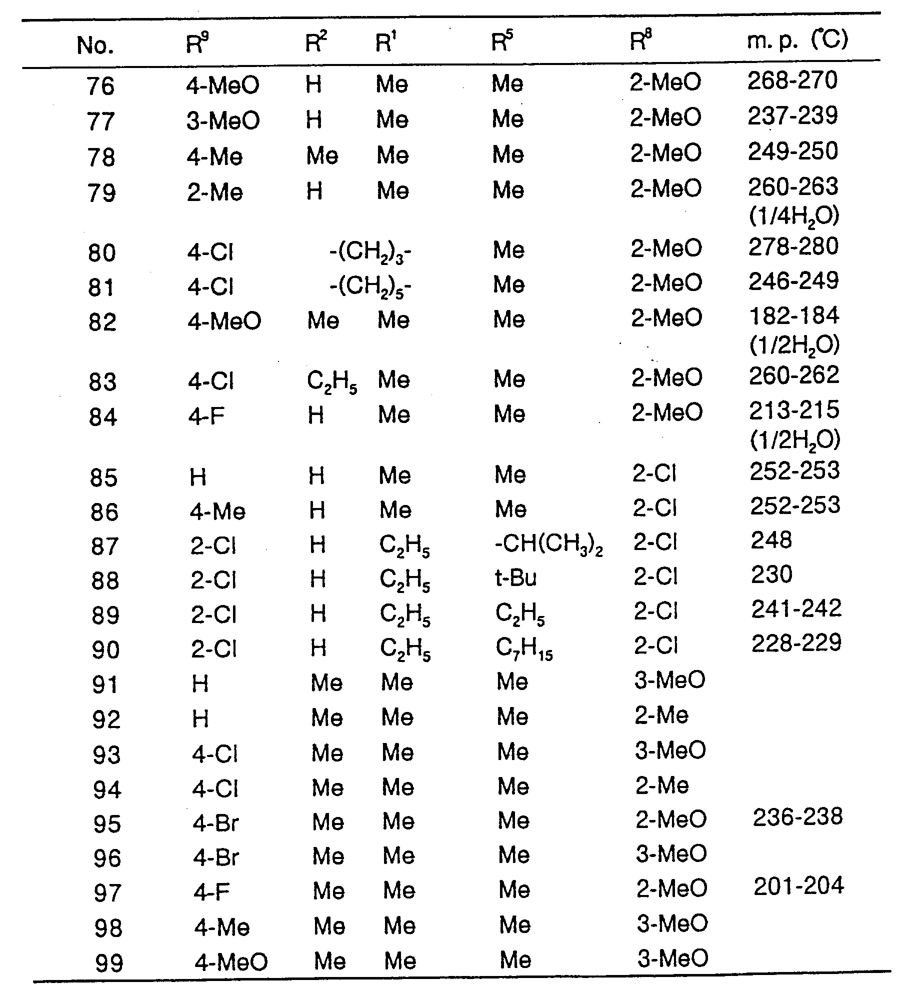

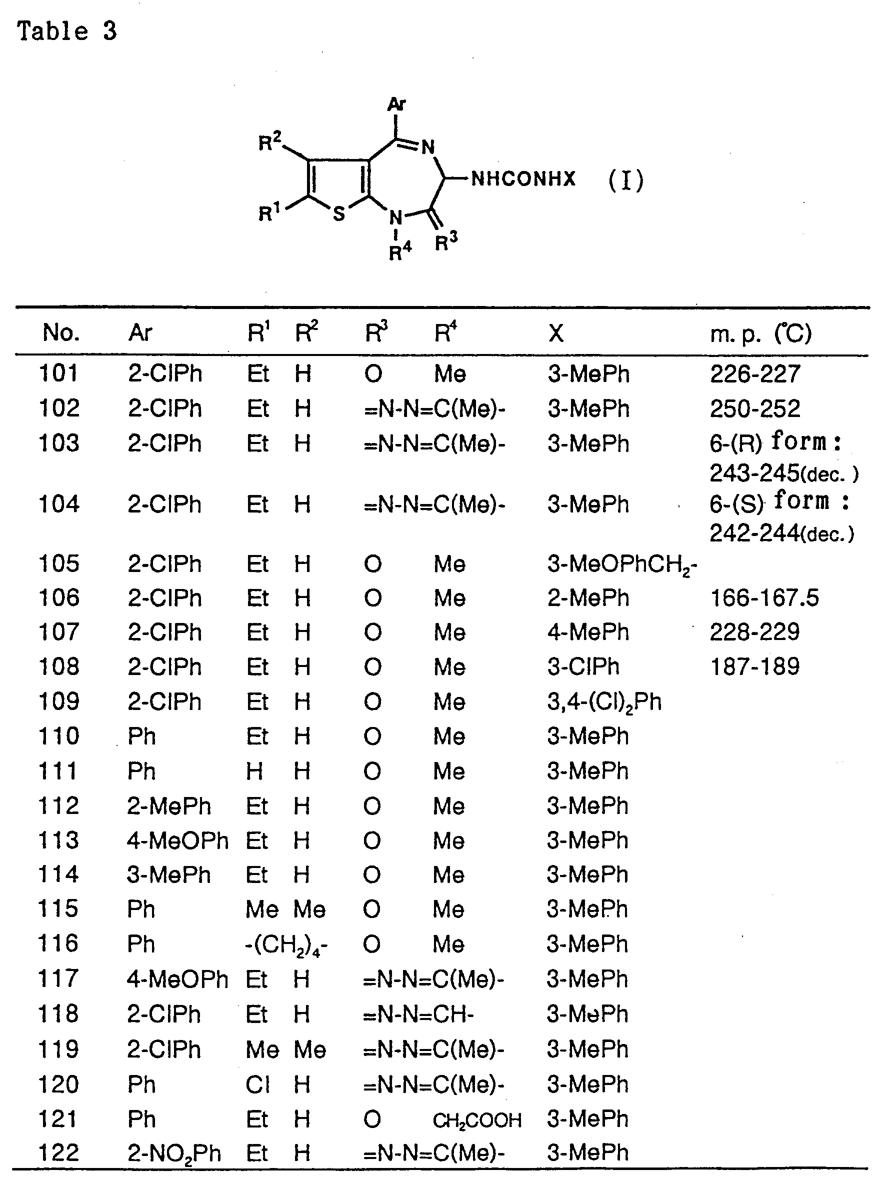

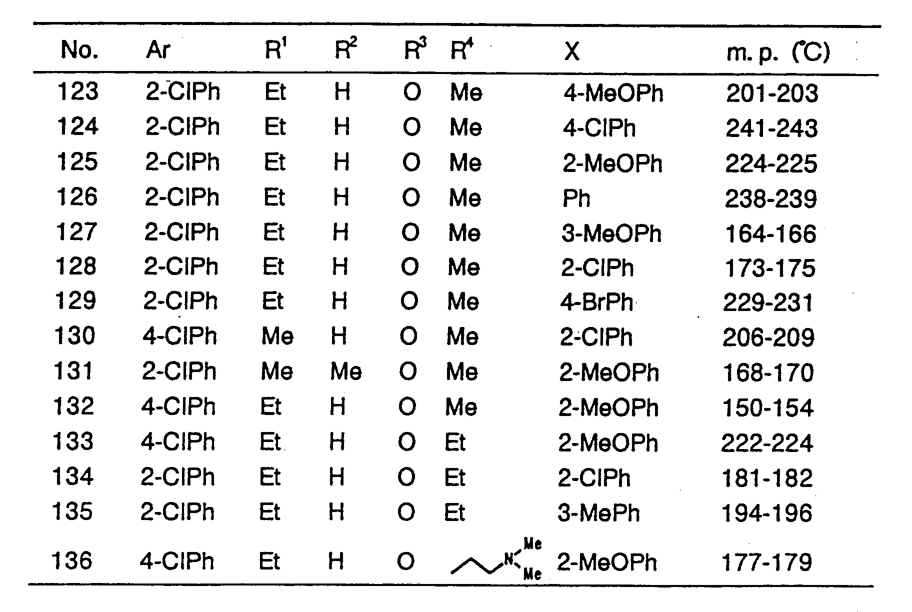

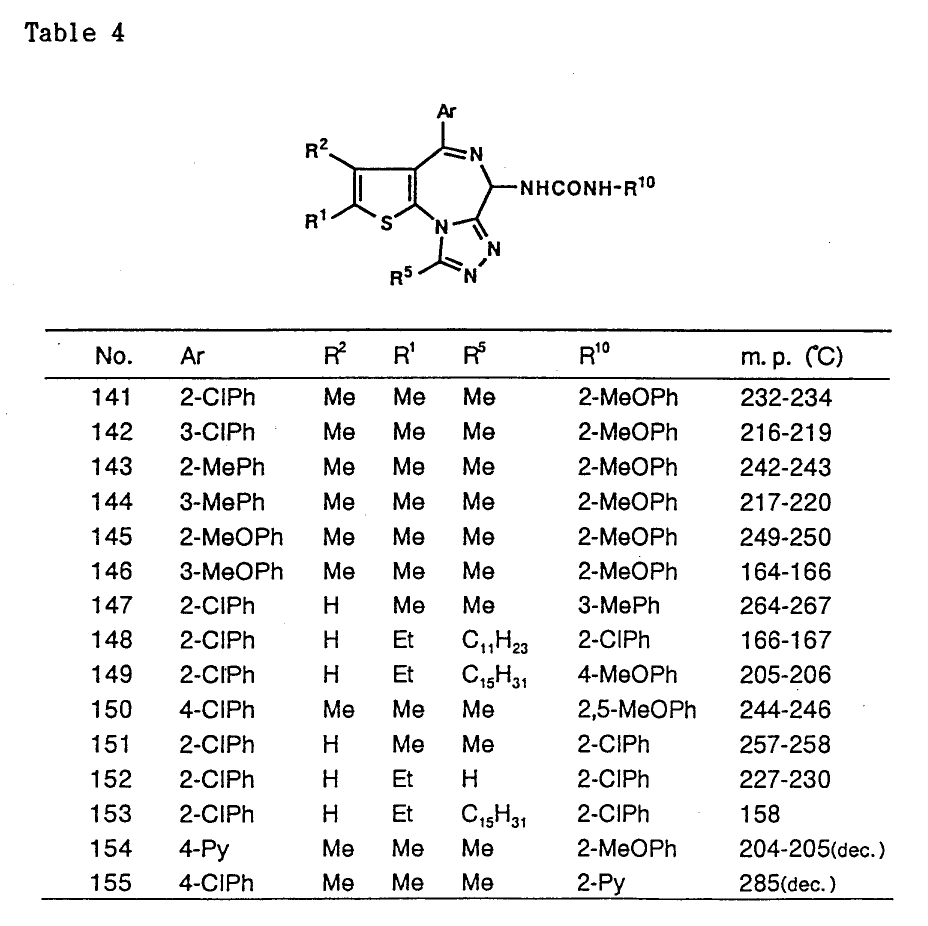

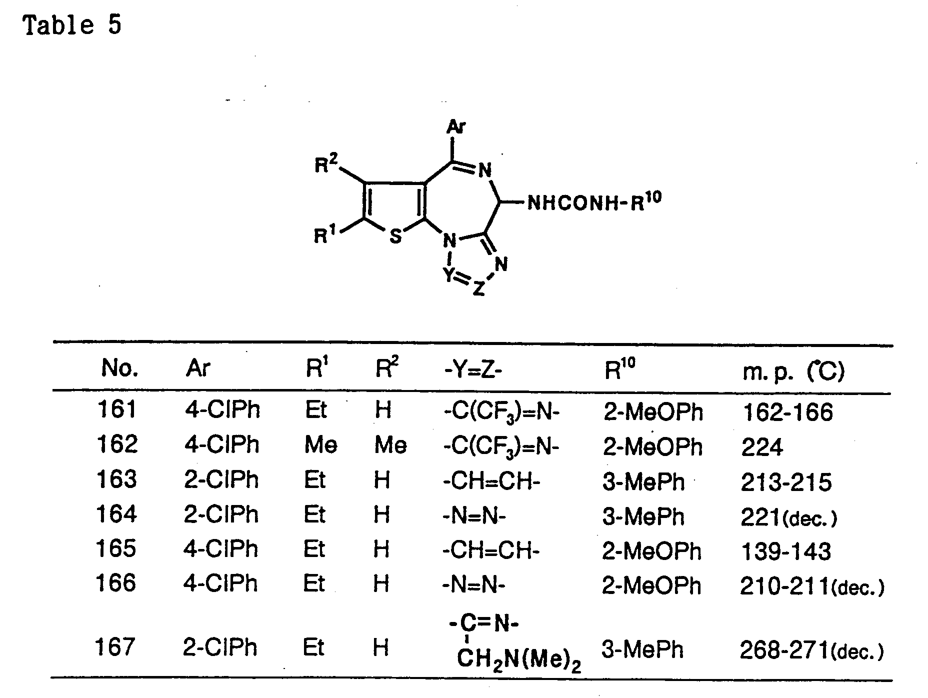

When preparing a medicine of the present invention, a compound of the formula (I) or (II) is preferably contained in a therapeutically effective amount, which is generally about 0.1-50 mg. Said medicine containing, besides the effective ingredient, pharmaceutically acceptable carrier, excipient, diluent, solubilizer, disintegrator, binder and the like, is formulated into tablet, powder, capsule, injection, suppository, transfusion and the like, and can be administered orally or parenterally to patients safely. The dosage is generally about 5-100 mg by oral administration and about 1-50 mg by intravenous administration daily for an adult.

### Best Mode for Embodying the Invention

The present invention is explained in more detail in the following by reference to Formulation Examples, Experimental Examples, Starting Material Preparation Examples and Examples. Needless to say, the present invention is not limited to these Examples.

### Formulation Example 1

The compound of Example 1 (0.5 part), lactose (25 parts), crystalline cellulose (35 parts) and corn starch (3 parts) were thoroughly mixed, and the mixture was kneaded well with a binder prepared from 2 parts of corn starch. The kneaded mixture was passed through a 16 mesh sieve, dried in an oven at 50°C and passed through a 24 mesh sieve. The thus-obtained kneaded powder was mixed well with corn starch (8 parts), crystalline cellulose (11 parts) and talc (9 parts), and compressed with pressure to give tablets containing 0.5 mg of the active ingredient per tablet.

### Formulation Example 2

The compound of Example 1 (1.0 mg) and sodium chloride (9.0 mg) were dissolved in an injectable water and filtered to remove pyrogen. The filtrate was aseptically placed in an ampoule, sterilized and melt-sealed to give an injection containing 1.0 mg of the active ingredient.

### Experimental Example 1: Effect on CD11b expression in human histiocytic leukemia cell line, U937

Effect on the expression of CD11b antigen, which constitutes the α chain of Mac-1, one of the adhesion molecules expressing on the surface of leukocytic cells, was investigated. U937 cells were suspended in RPMI1640 medium containing 20% fetal calf serum, plated with phorbol 12,13-dibutyrate (PDB, 10 ng/ml) and test compound into 96-well filtration plates (30,000 cells/well), and cultured for 3 days at 37°C in 5% CO₂. After the cells were washed once with RPMI1640 medium, rat anti-human CD11b monoclonal antibody (2 µg/ml) was added and the plates were stood for 1 hour on ice. After the cells were washed twice, peroxidase-conjugated anti-rat immunoglobulin antibody (1 µg/ml) was added, and the cells were stood for 1 hour on ice. After the cells were washed 3 times, color development substrate for peroxidase (o-phenylenediamine) was added. The plates were allowed to stand for 15 minutes at room temperature, and absorbance at 490 nm was measured with a 96-well plate reader. The absorbance was regarded as an indicator of CD11b antigen expression.

Table 1 shows IC₅₀ values (µM) obtained by calculating % inhibition when the absorbance in the presence and absence of PDB was taken as 100% and 0%, respectively.

**Table 6**

| Test Compound | Inhibitory effect on CD11b expression (IC₅₀, µM) |
|---|---|
| Example 1 | 0.25 |
| Example 2 | 0.25 |
| Example 3 | 0.20 |
| Example 4 | 0.87 |
| Example 9 | 0.43 |
| Example 11 | 0.44 |
| Example 17 | 0.21 |
| Example 20 (-) form | 0.063 |
| Example 21 | 0.33 |
| Example 22 | 0.47 |
| Example 23 | 0.33 |
| Example 24 | 0.21 |

As shown in Table 6 above, the compounds of the present invention strongly inhibited CD11b expression in U937 cells.

### Experimental Example 2: Effect on cell adhesion of guinea pig peritoneal cells to human umbilical vein-derived endothelial cells (HUVECs)

HUVECs were suspended in 199 medium containing 20% fetal calf serum, 20 µg/ml endothelial cell growth factor derived from bovine brain and 100 µg/ml heparin, plated into 96-well microculture plates coated with collagen, and cultured at 37°C in 5% CO₂. When cells were grown to confluence, interleukin 1 (10 U/ml) was added, and the cells were cultured for 24 hours. After the cells were washed once with 199 medium, leukotriene B4 (1 µM), test Compound A (compound of Example 1, hereinafter the same) and guinea pig peritoneal cells [300,000 cells/well, obtained by intraperitoneally injecting 0.1% oyster glycogen (25 ml) and recovering 5 hours later] were added, and the cells were incubated for 30 minutes at 37°C in 5% CO₂. After nonadherent cells were removed by inverting the plates, rose bengal solution (0.25% in phosphate-buffered physiological saline) was added and the plates were stood for 5 minutes. The cells were washed twice with 199 medium. Ethanol (50% in phosphate-buffered physiological saline) was added (200 µl/well) and the cells were stood for 30 minutes to allow leaking out of rose bengal dye incorporated into the cells. Absorbance at 520 nm was measured with a 96-well plate reader and the value obtained by subtracting absorbance of the well containing HUVECs alone was taken as an indicator of cell adhesion. Table 7 shows the result.

**Table 7**

| Leukotriene B4 (µM) | Compound A (µM) | Cell adhesion (absorbance at 520 nm) | Inhibition (%) |
|---|---|---|---|
| 0 | 0 | 0.074 | 100 |
| 1 | 0 | 0.214 | 0 |
| 1 | 0.01 | 0.222 | -4.7 |
| 1 | 0.1 | 0.169 | 32.6 |
| 1 | 1 | 0.080 | 32.6 |
| 1 | 1 | 0.080 | 95.5 |
| 1 | 10 | 0.077 | 97.9 |

As shown in Table 7, Compound A inhibited adhesion of guinea pig peritoneal cells to vascular endothelial cells in a concentration-dependent manner, and the concentration necessary for 50% inhibition (IC₅₀ values, µM) was about 0.2 µM.

### Experiment 3: Effect on oxazolone-induced ear edema in mice

Mice were sensitized by applying 50 µl of oxazolone solution (50 mg/ml in acetone) to their shaved abdomen. On day 7, 5 µl of oxazolone was applied to each surface of the right ear. After 24 hours, the right and left ears were cut out with a puncher of 6 mm in diameter and weighed with an electronic reading balance. The difference in weight between the treated ear (right) and untreated ear (left) was taken to reflect the degree of ear edema. Test Compound A was suspended in 0.5% methylcellulose solution and administered orally (0.1 ml/10 g body weight) on days 0 to 8. Table 8 shows the result.

**Table 8**

| Treatment | Compound A (mg/kg/day) | Ear edema (right ear-left ear) (mg, mean±S.D.) | Significance* |
|---|---|---|---|
| Unsensitized | 0 | 8.2±0.8 | |
| Sensitized | 0 | 17.6±1.1 | Control |
| Sensitized | 10 | 16.0±0.7 | P<0.01 |
| Sensitized | 30 | 15.4±0.5 | P<0.01 |
| Sensitized | 100 | 14.4±0.5 | P<0.01 |

| | | | |
|---|---|---|---|
| * : by Duncan's new multiple comparison test | | | |

As shown in Table 8, Compound A (Example 1) inhibited ear edema in mice in a dose-dependent manner, and this result suggested that Compound A suppressed infiltration of leukocytes into inflammatory sites by inhibiting cell adhesion *in vivo.*

### Starting Material Preparation Example 1

4-(4-Chlorophenyl)-2,3,9-trimethyl-6H-thieno[3,2-f][1,2,4]triazolo[4,3-a][1,4]diazepine (12 g) and sodium hydride (2.4 g) are added to diethyl carbonate (150 ml), and the mixture is heated. Bubbling of hydrogen gas begins at about 100°C and the reaction mixture gradually assumes purple. After refluxing for an hour, the reaction mixture is cooled to 20°C, added with O-(2,4-dinitrophenyl)hydroxylamine (8 g), and stirred for 2 hours. After the reaction, the reaction mixture is poured into ice water and a diethyl carbonate layer is partitioned. The layer is washed twice with water and dried over anhydrous magnesium sulfate. Diethyl carbonate is distilled away under reduced pressure and diisopropyl ether is added to the obtained residue. The precipitated crystals are filtrated to give 11 g of (6-amino-4-(4-chlorophenyl)-2,3,9-trimethyl-6H-thieno[3,2-f][1,2,4]triazolo[4,3-a][1,4]diazepin-6-yl)carboxylate.

Ethyl (6-amino-4-(4-chlorophenyl)-2,3,9-trimethyl-6H-thieno[3,2-f][1,2,4]triazolo[4,3-a][1,4]diazepin-6-yl)carboxylate (11 g) is dissolved in a mixed solution of ethanol (250 ml) and water (80 ml). Barium hydroxide 8 hydrate (8.4 g) is added and the mixture is stirred at room temperature for 24 hours. The solvent is distilled away under reduced pressure, and water (100 ml) is added. The mixture is adjusted to pH 2 with 1N hydrochloric acid and stirred for 20 minutes. The mixture is neutralized with an aqueous solution of sodium hydrogencarbonate and extracted twice with chloroform. The organic layer is dried over anhydrous magnesium sulfate. The solvent is distilled away under reduced pressure and the residue is subjected to silica gel column chromatography. The solvent is distilled away, the resulting residue is crystallized from diisopropyl ether, and the crystals are filtrated to give 7 g of 6-amino-4-(4-chlorophenyl)-2,3,9-trimethyl-6H-thieno[3,2-f][1,2,4]triazolo[4,3-a][1,4]diazepine.

### Starting Material Preparation Example 2

In the same manner as in Starting Material Preparation Example 1, 6-amino-2,3,9-trimethyl-4-phenyl-6H-thieno[3,2-f][1,2,4]triazolo[4,3-a][1,4]diazepine was obtained from 2,3,9-trimethyl-4-phenyl-6H-thieno[3,2-f][1,2,4]triazolo[4,3-a][1,4]diazepine.

### Starting Material Preparation Example 3

In the same manner as in Starting Material Preparation Example 1, 6-amino-2,3,9-trimethyl-4-(4-methylphenyl)-6H-thieno[3,2-f][1,2,4]triazolo[4,3-a][1,4]diazepine was obtained from 2,3,9-trimethyl-4-phenyl-6H-thieno[3,2-f]1,2,4]triazolo[4,3-a][1,4]diazepine.

### Starting Material Preparation Example 4

In the same manner as in Starting Material Preparation Example 1, 6-amino-4-(4-methoxyphenyl)-2,3,9-trimethyl-6H-thieno[3,2-f][1,2,4]triazolo[4,3-a][1,4]diazepine was obtained from 4-(4-methoxyphenyl)-2,3,9-trimethyl-6H-thieno[3,2-f][1,2,4]triazolo[4,3-a][1,4]diazepine.

### Example 1

6-Amino-4-(4-chlorophenyl)-2,3,9-trimethyl-6H-thieno[3,2-f][1,2,4]triazolo[4,3-a][1,4]diazepine (4 g) is dissolved in chloroform (30 ml). 2-Methoxyphenyl isocyanate (1.7 ml) is added, and the mixture is stirred for 30 minutes. The reaction mixture is subjected to silica gel column chromatography, and the resultant crystals are recrystallized from ethanol to give 5.14 g of N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea, melting point 261-262°C.

### Example 2

In the same manner as in Example 1, N-(2,3,9-trimethyl-4-phenyl-6H-thieno[3,2-f][1,2,4]triazolo[4,3-a][1,4]diazepin-6-yl)-N'-(2-methoxyphenyl)urea, melting point 191-194°C, was obtained from 6-amino-2,3,9-trimethyl-4-phenyl-6H-thieno[3,2-f][1,2,4]triazolo[4,3-a][1,4]diazepine and (2-methoxyphenyl)isocyanate.

### Example 3

In the same manner as in Example 1, N-(2,3,9-trimethyl-4-(4-methylphenyl)-6H-thieno[3,2-f][1,2,4]triazolo[4,3-a][1,4]-diazepin-6-yl)-N'-(2-methoxyphenyl)urea, melting point 249-250°C, was obtained from 6-amino-2,3,9-trimethyl-4-(4-methylphenyl)-6H-thieno[3,2-f][1,2,4]triazolo[4,3-a][1,4]diazepine and (2-methoxyphenyl)isocyanate.

### Example 4

In the same manner as in Example 1, N-(4-(4-methoxyphenyl)2,3,9-trimethyl-6H-thieno[3,2-f][1,2,4]triazolo[4,3-a][1,4]diazepin-6-yl)-N'-(2-methoxyphenyl)urea, melting point 182-184°C, was obtained from 6-amino-4-(4-methoxyphenyl)-2,3,9-trimethyl-6H-thieno[3,2-f][1,2,4]triazolo[4,3-a][1,4]diazepine and (2-methoxyphenyl)isocyanate.

In the same manner as in Example 1, the following compounds are obtained.

### Example 5

N-(2,3,9-Trimethyl-4-phenyl-6H-thieno[3,2-f][1,2,4]triazolo[4,3-a][1,4]diazepin-6-yl)-N'-(3-methoxyphenyl)urea

### Example 6

N-(2,3,9-Trimethyl-4-phenyl-6H-thieno[3,2-f][1,2,4]triazolo[4,3-a][1,4]diazepin-6-yl)-N'-(2-methylphenyl)urea

### Example 7

N-(4-(4-Chlorophenyl)-2,3,9-trimethyl-6H-thieno[3,2-f][1,2,4]triazolo[4,3-a][1,4]diazepin-6-yl)-N'-(3-methoxyphenyl)urea

### Example 8

N-(4-(4-Chlorophenyl)-2,3,9-trimethyl-6H-thieno[3,2-f][1,2,4]triazolo[4,3-a][1,4]diazepin-6-yl)-N'-(2-methylphenyl)urea

### Example 9

N-(4-(4-Bromophenyl)-2,3,9-trimethyl-6H-thieno[3,2-f][1,2,4]triazolo[4,3-a][1,4]diazepin-6-yl)-N'-(2-methoxyphenyl)urea, melting point 236-238°C

### Example 10

N-(4-(4-Bromophenyl)-2,3,9-trimethyl-6H-thieno[3,2-f][1,2,4]triazolo[4,3-a][1,4]diazepin-6-yl)-N'-(3-methoxyphenyl) urea

### Example 11

N-(4-(4-Fluorophenyl)-2,3,9-trimethyl-6H-thieno[3,2-f][1,2,4]triazolo[4,3-a][1,4]diazepin-6-yl)-N'-(2-methoxyphenyl)urea, melting point 201-204°C

### Example 12

N-(2,3,9-Trimethyl-4-(4-methylphenyl)-6H-thieno[3,2-f][1,2,4]triazolo[4,3-a][1,4]diazepin-6-yl)-N'-(3-methoxyphenyl)urea

### Example 13

N-4-(4-Methoxyphenyl)-2,3,9-trimethyl-6H-thieno[3,2-f][1,2,4]triazolo[4,3-a][1,4]diazepin-6-yl)-N'-(3-methoxyphenyl)urea

### Example 14

N-4-(2-Chlorophenyl)-2,3,9-trimethyl-6H-thieno[3,2-f][1,2,4]triazolo[4,3-a][1,4]diazepin-6-yl)-N'-(2-methoxyphenyl)urea, melting point 232-234°C

### Example 15

N-(4-(3-Chlorophenyl)-2,3,9-trimethyl-6H-thieno[3,2-f][1,2,4]triazolo[4,3-a][1,4]diazepin-6-yl)-N'-(2-methoxyphenyl)urea, melting point 216-219°C

### Example 16

N-(2,3,9-Trimethyl-4-(2-methylphenyl)-6H-thieno[3,2-f][1,2,4]triazolo[4,3-a][1,4]diazepin-6-yl)-N'-(2-methoxyphenyl)urea, melting point 242-243°C

### Example 17

N-(2,3,9-Trimethyl-4-(3-methylphenyl)-6H-thieno[3,2-f][1,2,4]triazolo[4,3-a][1,4]diazepin-6-yl)-N'-(2-methoxyphenyl)urea, melting point 217-220°C

### Example 18

N-(4-(2-Methoxyphenyl)-2,3,9-trimethyl-6H-thieno[3,2-f][1,2,4]triazolo[4,3-a][1,4]diazepin-6-yl)-N'-(2-methoxyphenyl)urea, melting point 249-250°C

### Example 19

N-(4-(3-Methoxyphenyl)-2,3,9-trimethyl-6H-thieno[3,2-f][1,2,4]triazolo[4,3-a][1,4]diazepin-6-yl)-N'-(2-methoxyphenyl)urea, melting point 164-166°C

### Example 20

N-(4-(4-Chlorophenyl)-2,3,9-trimethyl-6H-thieno[3,2-f][1,2,4]triazolo[4,3-a][1,4]diazepin-6-yl)-N'-(2-methoxyphenyl)urea (200 mg) obtained in Example 1 was subjected to the separation by HPLC using an optical isomer separating column (Chirasphar, Merck, inner diameter 25 mm, length 250 mm) with a mixed solution of n-hexane:dioxane:isopropyl alcohol (volume ratio 1800:1200:45) as a mobile phase at a flow rate of 10 ml/minute. The fractions eluted out 40 minutes later were combined and concentrated under reduced pressure to give 53 mg of (+)-N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea as a white amorphous powder. [α]_{D}²⁴=+33.2° (c=0.82, methanol)

Then, the fractions eluted out 45 minutes later were concentrated under reduced pressure to give 60 mg of (-)-N-(4-(4-chlorophenyl)-2,3,9-trimethyl-6H-thieno[3,2-f][1,2,4] triazolo[4,3-a][1,4]diazepin-6-yl)-N'-(2-methoxyphenyl)urea as a white amorphous powder. [α]_{D}²³=-37.4° (c=0.6, methanol)

### Example 21

N-(6-(4-Chlorophenyl)-11-methyl-2,3,4,5-tetrahydro-8H-[1]benzothieno[3,2-f][1,2,4]triazolo[4,3-a][1,4]diazepin-8-yl)-N'-(2-methoxyphenyl)urea, melting point 255-257°C

### Example 22

N-(5-(4-Chlorophenyl)-3,4-dihydro-2H,7H-cyclopenta[4,5]thieno[3,2-f][1,2,4]triazolo[4,3-a][1,4]diazepin-7-yl)-N'-(2-methoxyphenyl)urea, melting point 278-280°C

### Example 23

N-(4-(4-Chlorophenyl)-2-ethyl-3,9-dimethyl-6H-thieno[3,2-f][1,2,4]triazolo[4,3-a][1,4]diazepin-6-yl)-N'-(2-methoxyphenyl)urea, melting point 249-251°C

### Example 24

N-(4-(4-Chlorophenyl)-2,9-dimethyl-6H-thieno[3,2-f][1,2,4]triazolo[4,3-a][1,4]diazepin-6-yl)-N'-(2-methoxyphenyl)urea, melting point 255-257°C

### Starting Material Preparation Example 5

4-(4-Chlorophenyl)-2-ethyl-6H-thieno[3,2-f][1,3]imidazolo[1,2-a][1,4]diazepine (2.85 g) and sodium hydride (0.59 g) are added to diethyl carbonate (80 ml), and the mixture is heated. Bubbling of hydrogen gas begins at about 100°C and the reaction mixture gradually assumes purple. After refluxing for an hour, the reaction mixture is cooled to 20°C, added with O-(2,4-dinitrophenyl)hydroxylamine (1.91 g), and stirred for 2 hours. After the reaction, the reaction mixture is poured into ice water and a diethyl carbonate layer is separated. The layer is washed twice with water and dried over anhydrous magnesium sulfate. Diethyl carbonate is distilled away under reduced pressure and the obtained residue is subjected to silica gel column chromatography using chloroform-methanol to give 2.4 g of (6-amino-4-(4-chlorophenyl)-2-ethyl-6H-thieno[3,2-f][1,3]imidazolo[1,2-a][1,4]diazepin-6-yl)carboxylate.

Ethyl (6-amino-4-(4-chlorophenyl)-2-ethyl-6H-thieno[3,2-f][1,3]imidazolo[1,2-a][1,4]diazepin-6-yl)carboxylate (2.4 g) is dissolved in ethanol (50 ml). 1N Sodium hydroxide (12 ml) is added and the mixture is stirred at room temperature for 3 hours. The solvent is distilled away under reduced pressure, and water (100 ml) is added. The mixture is adjusted to pH 4 with acetic acid and stirred for 1 hour. The mixture is neutralized with an aqueous solution of sodium hydrogencarbonate and extracted with chloroform. The organic layer is dried over anhydrous magnesium sulfate. The solvent is distilled away under reduced pressure and the residue is subjected to silica gel column chromatography to give 1.2 g of yellow amorphous 6-amino-4-(4-chlorophenyl)-2-ethyl-6H-thieno[3,2-f][1,3]imidazolo[1,2-a][1,4]diazepine.

### Starting Material Preparation Example 6

4-(4-Chlorophenyl)-2-ethyl-6H-thieno[3,2-f][1,2,3,4]tetrazolo[4,5-a][1,4]diazepine (3.5 g) and sodium hydride (0.72 g) are added to diethyl carbonate (100 ml), and the mixture is heated. Bubbling of hydrogen gas begins at about 100°C and the reaction mixture gradually assumes purple. After refluxing for an hour, the reaction mixture is cooled to 20°C, added with O-(2,4-dinitrophenyl)hydroxylamine (1.91 g), and stirred for 2 hours. After the reaction, the reaction mixture is poured into ice water and a diethyl carbonate layer is separated. The layer is washed twice with water and dried over anhydrous magnesium sulfate. Diethyl carbonate is distilled away under reduced pressure and the obtained residue is subjected to silica gel column chromatography using chloroform-methanol to give 2.5 g of ethyl (6-amino-4-(4-chlorophenyl)-2-ethyl-6H-thieno[3,2-f][1,2,3,4]tetrazolo[4,5-a][1,4]diazepin-6-yl)carboxylate as yellow crystals.

Ethyl (6-amino-4-(4-chlorophenyl)-2-ethyl-6H-thieno[3,2-f][1,2,3,4]tetrazolo[4,5-a][1,4]diazepin-6-yl)carboxylate (2.5 g) is dissolved in ethanol (50 ml). 1N Sodium hydroxide (9.3 ml) is added and the mixture is stirred at room temperature for 3 hours. The solvent is distilled away under reduced pressure, and water (100 ml) is added. The mixture is adjusted to pH 4 with acetic acid and stirred for 1 hour. The mixture is neutralized with an aqueous solution of sodium hydrogen-carbonate and extracted with chloroform. The organic layer is dried over anhydrous magnesium sulfate. The solvent is distilled away under reduced pressure and the residue is subjected to silica gel column chromatography to give 0.9 g of yellow amorphous 6-amino-4-(4-chlorophenyl)-2-ethyl-6H-thieno[3,2-f][1,2,3,4]tetrazolo[4,5-a][1,4]diazepine.

### Starting Material Preparation Example 7

In the same manner as in Starting Material Preparation Example 5, 6-amino-4-(2-chlorophenyl)-2-ethyl-6H-thieno[3,2-f][1,3]imidazolo[1,2-a][1,4]diazepine was obtained from 4-(2-chlorophenyl)-2-ethyl-6H-thieno[3,2-f][1,3]imidazolo[1,2-a][1,4]diazepine.

### Starting Material Preparation Example 8

In the same manner as in Starting Material Preparation Example 6, 6-amino-4-(2-chlorophenyl)-2-ethyl-6H-thieno[3,2-f][1,2,3,4]tetrazolo[4,5-a][1,4]diazepine was obtained from 4-(2-chlorophenyl)-2-ethyl-6H-thieno[3,2-f][1,2,3,4]tetrazolo[4,5-a][1,4]diazepine.

### Example 25

6-Amino-4-(4-chlorophenyl)-2-ethyl-6H-thieno[3,2-f][1,3]imidazolo[1,2-a][1,4]diazepine (0.5 g) was dissolved in chloroform (15 ml). 2-Methoxyphenyl isocyanate (0.2 ml) was added and the mixture was stirred at room temperature for an hour. The solvent was distilled away under reduced pressure and isopropyl ether-ethyl acetate was added to the obtained residue to give crystals. The crystals were recrystallized from isopropyl etherethyl acetate to give 0.22 g of N-(4-(4-chlorophenyl)-2-ethyl-6H-thieno[3,2-f][1,3]imidazolo[1,2-a][1,4]diazepin-6-yl)-N'-(2-methoxyphenyl)urea, melting point 139-143°C.

### Example 26

6-Amino-4-(4-chlorophenyl)-2-ethyl-6H-thieno[3,2-f][1,2,3,4]tetrazolo[4,5-a][1,4]diazepine (0.3 g) was dissolved in chloroform (15 ml). 2-Methoxyphenyl isocyanate (0.2 ml) was added and the mixture was stirred at room temperature for an hour. The solvent was distilled away under reduced pressure and isopropyl ether was added to the obtained residue to give crystals. The crystals were recrystallized from methanol to give 0.06 g of N-(4-(4-chlorophenyl)-2-ethyl-6H-thieno[3,2-f][1,2,3,4]tetrazolo[4,5-a][1,4]diazepin-6-yl)-N'-(2-methoxyphenyl)urea, melting point 210-211°C (decomposition).

### Example 27

In the same manner as in Example 25, N-(4-(2-chlorophenyl)2-ethyl-6H-thieno[3,2-f][1,2,3,4]tetrazolo[4,5-a][1,4]diazepin-6-yl)-N'-(3-methylphenyl)urea, melting point 213-215°C, was obtained from 6-amino-4-(2-chlorophenyl)-2-ethyl-6H-thieno[3,2-f][1,3]imidazolo[1,2-a][1,4]diazepine and 3-methylphenyl isocyanate.

### Example 28

In the same manner as in Example 26, N-(4-(2-chlorophenyl)2-ethyl-6H-thieno[3,2-f][1,2,3,4]tetrazolo[4,5-a][1,4]diazepin-6-yl)-N'-(3-methylphenyl)urea, melting point 221°C (decomposition), was obtained from 6-amino-4-(2-chlorophenyl)-2-ethyl-6H-thieno[3,2-f][1,2,3,4]tetrazolo[4,5-a][1,4]diazepine and 3-methylphenyl isocyanate.

In the same manner as in the above-mentioned Examples, the compounds shown in Table 2 to Table 5 supra were obtained.

### Industrial Applicability

As is evident from the Experimental Examples as described, the thieno(triazolo)diazepine compound of the present invention has an inhibitory effect on leukocyte adhesion to human umbilical vein-derived endothelial cells by inhibiting the CD11b expression on leukocytes, an inhibitory effect on the adhesion of guinea pig peritoneal cells to human umbilical vein-derived endothelial cells (HUVEC), and an inhibitory effect on leukocyte filtration *in vivo*. These effects suggest that the compound of the present invention is useful as a therapeutic or prophylactic drug for various inflammatory diseases, allergic diseases, rheumatoid arthritis and so on, in which cell adhesion is involved in the onset and progress thereof. In addition, the compound of the present invention can be used for preventing or treating autoimmune diseases and graft rejection in organ transplantation, and for preventing metastasis of malignant cells.

## Claims

1. A cell adhesion inhibitor comprising, as an active ingredient, a thienotriazolodiazepine compound of the formula 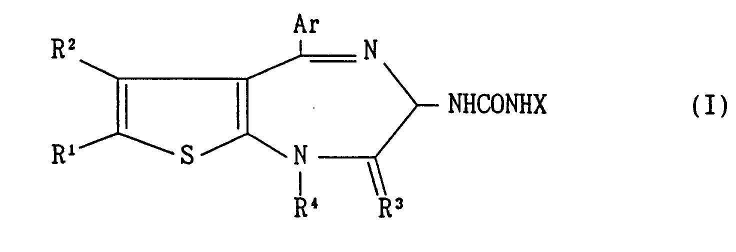 wherein
R¹ and R² are the same or different, and each is a hydrogen, a halogen, an alkyl or an aralkyl, or R¹ and R² bind with each other to form a ring;
R³ is an oxygen;
R⁴ is a hydrogen, an alkyl, an alkenyl or a group of the formula: -(CH₂)mCOOR⁶ where R⁶ is a hydrogen, an alkyl, an alkenyl or an aralkyl, and m is an integer of 1 to 6, or
R³ and R⁴ bind with each other to form a group of the formula: =N-N=C(R⁵)- where R⁵ is a hydrogen, an alkyl, an alkenyl or an aralkyl, or a group of the formula: -(CH₂)nCOOR⁷ where R⁷ is a hydrogen, an alkyl, an alkenyl or an aralkyl and n is an integer of 1 to 6; and
Ar and X are the same or different and each is an aryl, an aralkyl or a heteroaryl,
or a pharmaceutically acceptable salt thereof.

2. A thienodiazepine compound of the formula 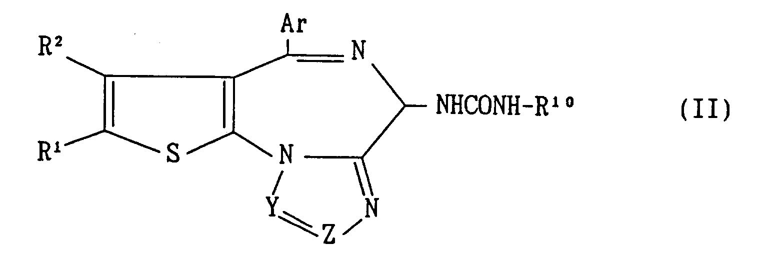 wherein
Ar is a phenyl, a pyridyl, a phenyl having, as a substituent, halogen, alkyl having 1 to 4 carbon atoms, alkoxy having 1 to 4 carbon atoms, cyano, amino, nitro or trihalomethyl, or a pyridyl having, as a substituent, halogen, alkyl having 1 to 4 carbon atoms or alkoxy having 1 to 4 carbon atoms;
R¹ and R² are the same or different and each is a hydrogen, a halogen or an alkyl, or R¹ and R² bind with each other to form a ring;
-Y=Z- is a group of the formula: -N=N-, -C(R⁵)=N- where R⁵ is a hydrogen, a halogen, an alkyl, a haloalkyl or a group of the formula: -(CH₂)nNR¹¹R¹² where R¹¹ and R¹² are the same or different and each is a hydrogen, an alkyl having 1 to 4 carbon atoms, an alkenyl having 2 to 8 carbon atoms or an aralkyl and n is an integer of 1 to 6, or -CH=CH-; and
R¹⁰ is a phenyl, a pyridyl or a phenyl having 1 to 3 substituents selected from the group of halogen, amino, nitro, trihalomethyl, alkyl having 1 to 4 carbon atoms and alkoxy having 1 to 4 carbon atoms,
an optically active compound thereof or a pharmaceutically acceptable salt thereof.

3. The compound of Claim 2, which is a thienotriazolodiazepine compound of the formula 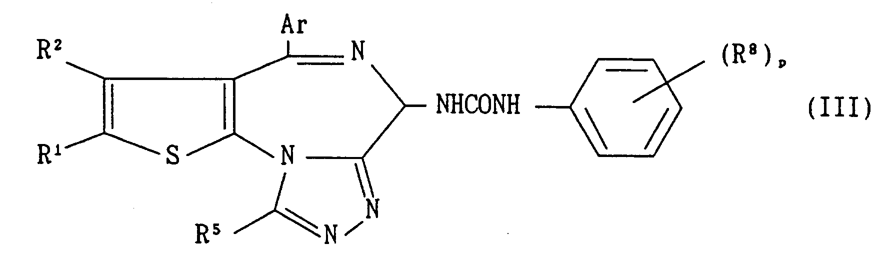 wherein
Ar is a phenyl, a pyridyl, a phenyl having, as a substituent, halogen, alkyl having 1 to 4 carbon atoms, alkoxy having 1 to 4 carbon atoms, cyano, amino, nitro or trihalomethyl, or a pyridyl having, as a substituent, halogen, alkyl having 1 to 4 carbon atoms or alkoxy having 1 to 4 carbon atoms;
R¹ and R² are the same or different and each is a hydrogen, a halogen or an alkyl having 1 to 4 carbon atoms, or R¹ and R² bind with each other to form a ring;
R⁵ is a hydrogen, a halogen or an alkyl having 1 to 4 carbon atoms;
R⁸ is a hydrogen, a halogen, an amino, a nitro, a trihalomethyl, an alkyl having 1 to 4 carbon atoms or an alkoxy having 1 to 4 carbon atoms; and
p is 1, 2 or 3, provided that when p is 2 or 3, the groups represented by R⁸ are the same or different,
an optically active compound thereof or a pharmaceutically acceptable salt thereof.

4. The compound of Claim 2, wherein the compound of the formula (II) is a thienotriazolodiazepine compound of the formula 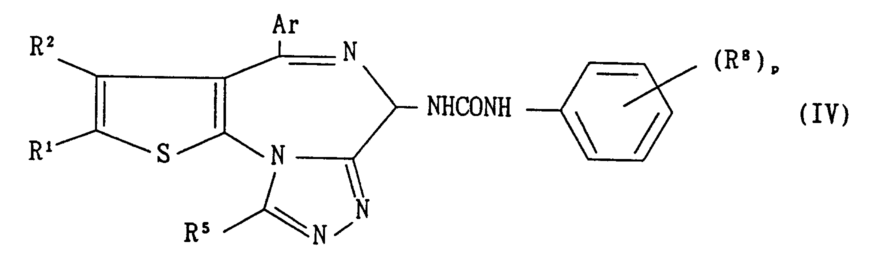 wherein
Ar is a phenyl or a phenyl having, as a substituent, halogen, alkyl having 1 to 4 carbon atoms or alkoxy having 1 to 4 carbon atoms;
R¹ is an alkyl having 1 to 4 carbon atoms;
R² is a hydrogen or an alkyl having 1 to 4 carbon atoms; or R¹ and R² bind with each other to form a 5- to 7-membered ring;
R⁵ is an alkyl having 1 to 4 carbon atoms;
R⁸ is an alkyl having 1 to 4 carbon atoms or an alkoxy having 1 to 4 carbon atoms; and
p is 1 or 2, provided that when p is 2, the groups represented by R⁸ are the same or different,
an optically active compound thereof or a pharmaceutically acceptable salt thereof.

5. The compound of Claim 2, wherein the compound of the formula (II) is a thienotriazolodiazepine compound of the formula 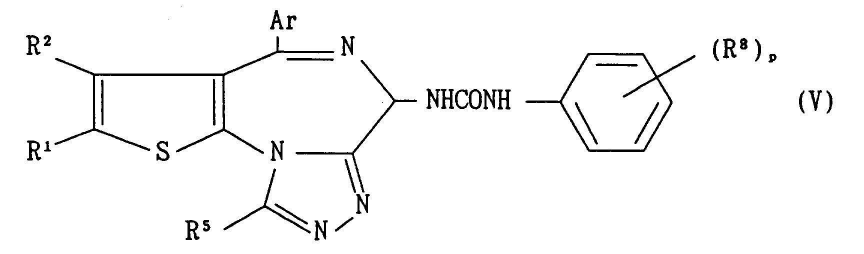 wherein
Ar is a phenyl or a phenyl having, as a substituent, halogen, methyl or methoxy;
R¹ is a methyl or an ethyl;
R² is a hydrogen or a methyl; or R¹ and R² bind with each other to form a group of the formula: -(CH₂)₃- or -(CH₂)₄-;
R⁵ is a methyl;
R⁸ is a methyl or a methoxy; and
p is 1 or 2, provided that when p is 2, the groups represented by R⁸ are the same or different,
an optically active compound thereof or a pharmaceutically acceptable salt thereof.

6. The compound of Claim 2, wherein the compound of the formula (II) is a thienotriazolodiazepine compound of the formula 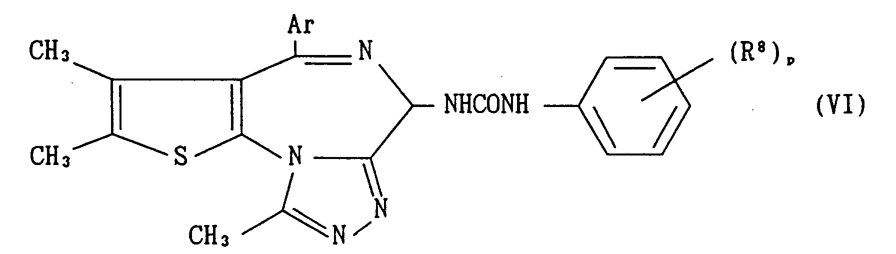 wherein
Ar is a phenyl or a phenyl having, as a substituent, halogen, methyl or methoxy;
R⁸ is a methyl or a methoxy; and
p is 1 or 2, provided that when p is 2, the groups represented by R⁸ are the same or different,
an optically active compound thereof or a pharmaceutically acceptable salt thereof.

7. The compound of Claim 2, wherein the compound of the formula (II) is a member selected from the group consisting of
(1) N-4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(2) N-(2,3,9-trimethyl-4-phenyl-6H-thieno[3,2-f][1,2,4]triazolo[4,3-a][1,4]diazepin-6-yl)-N'-(2-methoxyphenyl)urea,
(3) N-(2,3,9-trimethyl-4-(4-methylphenyl)-6H-thieno[3,2-f][1,2,4]triazolo[4,3-a][1,4]diazepin-6-yl)-N'-(2-methoxyphenyl)urea,
(4) N-(4-(4-methoxyphenyl)-2,3,9-trimethyl-6H-thieno[3,2-f][1,2,4]triazolo[4,3-a][1,4]diazepin-6-yl)-N'-(2-methoxyphenyl)urea,
(5) N-(2,3,9-trimethyl-4-phenyl-6H-thieno[3,2-f][1,2,4]triazolo[4,3-a][1,4]diazepin-6-yl)-N'-(3-methoxyphenyl)urea,
(6) N-(2,3,9-trimethyl-4-phenyl-6H-thieno[3,2-f][1,2,4]triazolo[4,3-a][1,4]diazepin-6-yl)-N'-(2-methylphenyl)urea,
(7) N-(4-(4-chlorophenyl)-2,3,9-trimethyl-6H-thieno[3,2-f][1,2,4]triazolo[4,3-a][1,4]diazepin-6-yl)-N'-(3-methoxyphenyl)urea,
(8) N-(4-(4-chlorophenyl)-2,3,9-trimethyl-6H-thieno[3,2-f][1,2,4]triazolo[4,3-a][1,4]diazepin-6-yl)-N'-(2-methylphenyl)urea,
(9) N-(4-(4-bromophenyl)-2,3,9-trimethyl-6H-thieno[3,2-f][1,2,4]triazolo[4,3-a][1,4]diazepin-6-yl)-N'-(2-methoxyphenyl)urea,
(10) N-(4-(4-bromophenyl)-2,3,9-trimethyl-6H-thieno[3,2-f][1,2,4]triazolo[4,3-a][1,4]diazepin-6-yl)-N'-(3-methoxyphenyl)urea,
(11) N-(4-(4-fluorophenyl)-2,3,9-trimethyl-6H-thieno[3,2-f][1,2,4]triazolo[4,3-a][1,4]diazepin-6-yl)-N'-(2-methoxyphenyl)urea,
(12) N-(2,3,9-trimethyl-4-(4-methylphenyl)-6H-thieno[3,2-f][1,2,4]triazolo[4,3-a][1,4]diazepin-6-yl)-N'-(3-methoxyphenyl)urea,
(13) N-4-(4-methoxyphenyl)-2,3,9-trimethyl-6H-thieno[3,2-f][1,2,4]triazolo[4,3-a][1,4]diazepin-6-yl)-N'-(3-methoxyphenyl)urea,
(14) N-4-(2-chlorophenyl)-2,3,9-trimethyl-6H-thieno[3,2-f][1,2,4]triazolo[4,3-a][1,4]diazepin-6-yl)-N'-(2-methoxyphenyl)urea,
(15) N-(4-(3-chlorophenyl)-2,3,9-trimethyl-6H-thieno[3,2-f][1,2,4]triazolo[4,3-a][1,4]diazepin-6-yl)-N'-(2-methoxy phenyl)urea,
(16) N-(2,3,9-trimethyl-4-(2-methylphenyl)-6H-thieno[3,2-f][1,2,4]triazolo[4,3-a][1,4]diazepin-6-yl)-N'-(2-methoxyphenyl)urea,
(17) N-(2,3,9-trimethyl-4-(3-methylphenyl)-6H-thieno[3,2-f][1,2,4]triazolo[4,3-a][1,4]diazepin-6-yl)-N'-(2-methoxyphenyl)urea,
(18) N-(4-(2-methoxyphenyl)-2,3,9-trimethyl-6H-thieno[3,2-f][1,2,4]triazolo[4,3-a][1,4]diazepin-6-yl)-N'-(2-methoxyphenyl)urea,
(19) N-(4-(3-methoxyphenyl)-2,3,9-trimethyl-6H-thieno[3,2-f][1,2,4]triazolo[4,3-a][1,4]diazepin-6-yl)-N'-(2-methoxyphenyl)urea,
(20) (+)-N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(21) (-)-N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(22) N-(4-(2-chlorophenyl)-2,3,9-trimethyl-6H-thieno[3,2-f][1,2,4]triazolo[4,3-a][1,4]diazepin-6-yl)-N'-(3-methylphenyl)urea,
(23) N-(4-(4-chlorophenyl)-2,3,9-trimethyl-6H-thieno[3,2-f][1,2,4]triazolo[4,3-a][1,4]diazepin-6-yl)-N'-(2,5-dimethoxyphenyl)urea,
(24) N-(6-(4-chlorophenyl)-11-methyl-2,3,4,5-tetrahydro-8H-[1]benzothieno[3,2-f][1,2,4]triazolo[4,3-a][1,4]diazepin-8- yl)-N'-(2-methoxyphenyl)urea,
(25) N-(5-(4-chlorophenyl)-3,4-dihydro-2H,7H-cyclopenta[4,5]thieno[3,2-f][1,2,4]triazolo[4,3-a][1,4]diazepin-7-yl)-N'-(2-methoxyphenyl)urea,
(26) N-(4-(4-chlorophenyl)-2-ethyl-3,9-dimethyl-6H-thieno[3,2-f][1,2,4]triazolo[4,3-a][1,4]diazepin-6-yl)-N'-(2-methoxyphenyl)urea, and
(27) N-(4-(4-chlorophenyl)-2,9-dimethyl-6H-thieno[3,2-f][1,2,4]triazolo[4,3-a][1,4]diazepin-6-yl)-N'-(2-methoxyphenyl)urea,
an optically active compound thereof or a pharmaceutically acceptable salt thereof.

8. The compound of Claim 2, wherein the compound of the formula (II) is a member selected from the group consisting of
(1) N-4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(2) N-(2,3,9-trimethyl-4-phenyl-6H-thieno[3,2-f][1,2,4]triazolo[4,3-a][1,4]diazepin-6-yl)-N'-(2-methoxyphenyl)urea,
(3) N-(2,3,9-trimethyl-4-(4-methylphenyl)-6H-thieno[3,2-f][1,2,4]triazolo[4,3-a][1,4]diazepin-6-yl)-N'-(2-methoxyphenyl)urea,
(4) N-(4-(4-methoxyphenyl)-2,3,9-trimethyl-6H-thieno[3,2-f][1,2,4]triazolo[4,3-a][1,4]diazepin-6-yl)-N'-(2-methoxyphenyl)urea,
(5) N-(2,3,9-trimethyl-4-phenyl-6H-thieno[3,2-f][1,2,4]triazolo[4,3-a][1,4]diazepin-6-yl)-N'-(3-methoxyphenyl)urea,
(6) N-(2,3,9-trimethyl-4-phenyl-6H-thieno[3,2-f][1,2,4]triazolo[4,3-a][1,4]diazepin-6-yl)-N'-(2-methylphenyl)urea,
(7) N-(4-(4-chlorophenyl)-2,3,9-trimethyl-6H-thieno[3,2-f][1,2,4]triazolo[4,3-a][1,4]diazepin-6-yl)-N'-(3-methoxyphenyl)urea,
(8) N-(4-(4-chlorophenyl)-2,3,9-trimethyl-6H-thieno[3,2-f][1,2,4]triazolo[4,3-a][1,4]diazepin-6-yl)-N'-(2-methylphenyl)urea,
(9) N-(4-(4-bromophenyl)-2,3,9-trimethyl-6H-thieno[3,2-f][1,2,4]triazolo[4,3-a][1,4]diazepin-6-yl)-N'-(2-methoxyphenyl)urea,
(10) N-(4-(4-bromophenyl)-2,3,9-trimethyl-6H-thieno[3,2-f][1,2,4]triazolo[4,3-a][1,4]diazepin-6-yl)-N'-(3-methoxyphenyl)urea,
(11) N-(4-(4-fluorophenyl)-2,3,9-trimethyl-6H-thieno[3,2-f][1,2,4]triazolo[4,3-a][1,4]diazepin-6-yl)-N'-(2-methoxyphenyl)urea,
(12) N-(2,3,9-trimethyl-4-(4-methylphenyl)-6H-thieno[3,2-f][1,2,4]triazolo[4,3-a][1,4]diazepin-6-yl)-N'-(3-methoxyphenyl)urea,
(13) N-4-(4-methoxyphenyl)-2,3,9-trimethyl-6H-thieno[3,2-f][1,2,4]triazolo[4,3-a][1,4]diazepin-6-yl)-N'-(3-methoxyphenyl)urea,
(14) N-4-(2-chlorophenyl)-2,3,9-trimethyl-6H-thieno[3,2-f][1,2,4]triazolo[4,3-a][1,4]diazepin-6-yl)-N'-(2-methoxy phenyl)urea,
(15) N-(4-(3-chlorophenyl)-2,3,9-trimethyl-6H-thieno[3,2-f][1,2,4]triazolo[4,3-a][1,4]diazepin-6-yl)-N'-(2-methoxyphenyl)urea,
(16) N-(2,3,9-trimethyl-4-(2-methylphenyl)-6H-thieno[3,2-f][1,2,4]triazolo[4,3-a][1,4]diazepin-6-yl)-N'-(2-methoxyphenyl)urea,
(17) N-(2,3,9-trimethyl-4-(3-methylphenyl)-6H-thieno[3,2-f][1,2,4]triazolo[4,3-a][1,4]diazepin-6-yl)-N'-(2-methoxyphenyl)urea,
(18) N-(4-(2-methoxyphenyl)-2,3,9-trimethyl-6H-thieno[3,2-f][1,2,4]triazolo[4,3-a][1,4]diazepin-6-yl)-N'-(2-methoxyphenyl)urea,
(19) N-(4-(3-methoxyphenyl)-2,3,9-trimethyl-6H-thieno[3,2-f][1,2,4]triazolo[4,3-a][1,4]diazepin-6-yl)-N'-(2-methoxyphenyl)urea,
(20) (+)-N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(21) (-)-N-(4-(4-chlorophenyl)-2,3,9-trimethyl-6H-thieno[3,2-f][1,2,4]triazolo[4,3-a][1,4]diazepin-6-yl)-N'-(2-methoxyphenyl)urea,
(22) N-(4-(2-chlorophenyl)-2,3,9-trimethyl-6H-thieno[3,2-f][1,2,4]triazolo[4,3-a][1,4]diazepin-6-yl)-N'-(3-methylphenyl)urea, and
(23) N-(4-(4-chlorophenyl)-2,3,9-trimethyl-6H-thieno[3,2-f][1,2,4]triazolo[4,3-a][1,4]diazepin-6-yl)-N'-(2,5-dimethoxyphenyl)urea,
an optically active compound thereof or a pharmaceutically acceptable salt thereof.

9. The compound of Claim 2, wherein the compound of the formula (II) is (-)-N-4-(4-chlorophenyl)-2,3,9-trimethyl-6H-thieno[3,2-f][1,2,4]triazolo[4,3-a][1,4]diazepin-6-yl)-N'-(2-methoxyphenyl)urea or a pharmaceutically acceptable salt thereof.

10. The compound of Claim 2, wherein the compound of the formula (II) is a compound of the formula 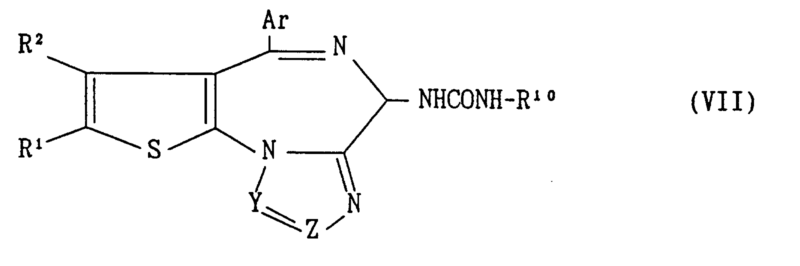 wherein
Ar is a phenyl, a pyridyl, a phenyl having, as a substituent, halogen, alkyl having 1 to 4 carbon atoms, alkoxy having 1 to 4 carbon atoms, cyano, amino, nitro or trihalomethyl, or a pyridyl having, as a substituent, halogen, alkyl having 1 to 4 carbon atoms or alkoxy having 1 to 4 carbon atoms;
R¹ and R² are the same or different and each is a hydrogen, a halogen or an alkyl having 1 to 4 carbon atoms, or R¹ and R² bind with each other to form a ring;
-Y=Z- is a group of the formula: -N=N-, -C(R⁵)=N- where R⁵ is a haloalkyl or a group of the formula: -(CH₂)nNR¹¹R¹² where R¹¹ and R¹² are the same or different and each is a hydrogen, an alkyl having 1 to 4 carbon atoms, an alkenyl having 2 to 8 carbon atoms or an aralkyl and n is an integer of 1 to 6, or -CH=CH-; and
R¹⁰ is a phenyl, a pyridyl or a phenyl having 1 to 3 substituents selected from halogen, amino, nitro, trihalomethyl, alkyl having 1 to 4 carbon atoms and alkoxy having 1 to 4 carbon atoms,
an optically active compound thereof or a pharmaceutically acceptable salt thereof.

11. A method for treating various diseases concerned with cell adhesion, comprising administering a thienodiazepine compound of the formula 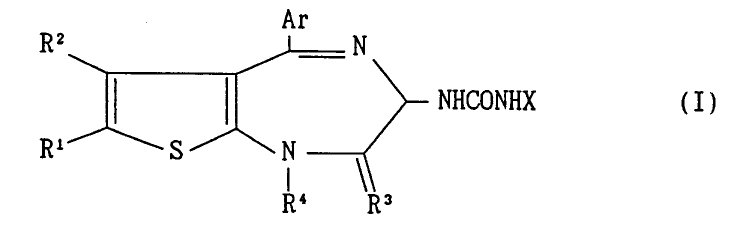 wherein
R¹ and R² are the same or different, and each is a hydrogen, a halogen, an alkyl or an aralkyl, or R¹ and R² bind with each other to form a ring;
R³ is an oxygen;
R⁴ is a hydrogen, an alkyl, an alkenyl or a group of the formula: -(CH₂)mCOOR⁶ where R⁶ is a hydrogen, an alkyl, an alkenyl or an aralkyl, and m is an integer of 1 to 6, or
R³ and R⁴ bind with each other to form a group of the formula: =N-N=C(R⁵)- where R⁵ is a hydrogen, an alkyl, an alkenyl or an aralkyl, or a group of the formula: -(CH₂)nCOOR⁷ where R⁷ is a hydrogen, an alkyl, an alkenyl or an aralkyl and n is an integer of 1 to 6; and
Ar and X are the same or different and each is an aryl, an aralkyl or a heteroaryl,
or a pharmaceutically acceptable salt thereof.

12. Use of a thienodiazepine compound of the formula 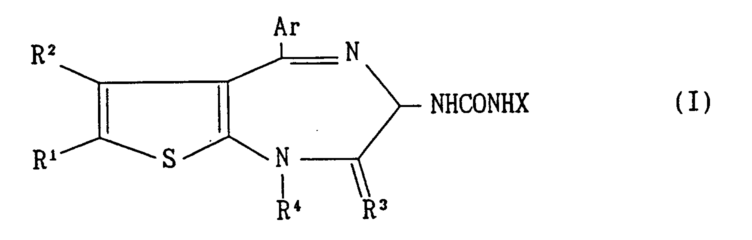 wherein
R¹ and R² are the same or different, and each is a hydrogen, a halogen, an alkyl or an aralkyl, or R¹ and R² bind with each other to form a ring;
R³ is an oxygen;
R⁴ is a hydrogen, an alkyl, an alkenyl or a group of the formula: -(CH₂)mCOOR⁶ where R⁶ is a hydrogen, an alkyl, an alkenyl or an aralkyl, and m is an integer of 1 to 6, or
R³ and R⁴ bind with each other to form a group of the formula: =N-N=C(R⁵)- where R⁵ is a hydrogen, an alkyl, an alkenyl or an aralkyl, or a group of the formula: -(CH₂)nCOOR⁷ where R⁷ is a hydrogen, an alkyl, an alkenyl or an aralkyl and n is an integer of 1 to 6; and
Ar and X are the same or different and each is an aryl, an aralkyl or a heteroaryl,
or a pharmaceutically acceptable salt thereof for producing a cell adhesion inhibitor.
